(19) Europäisches Patentamt
European Patent Office
Office européen des brevets

(11) EP 1 910 831 B1

(12) **EUROPÄISCHE PATENTSCHRIFT**

(45) Veröffentlichungstag und Bekanntmachung des Hinweises auf die Patenterteilung:
**08.01.2014 Patentblatt 2014/02**

(21) Anmeldenummer: **06778077.5**

(22) Anmeldetag: **31.07.2006**

(51) Int Cl.:
***C12Q 1/00*** *(2006.01)* ***G01N 27/48*** *(2006.01)*

(86) Internationale Anmeldenummer:
**PCT/EP2006/064842**

(87) Internationale Veröffentlichungsnummer:
**WO 2007/014931 (08.02.2007 Gazette 2007/06)**

(54) **VERFAHREN UND SYSTEM ZUR KONZENTRATIONSBESTIMMUNG EINES ANALYT-ENZYM-KOMPLEXES ODER ANALYT-ENZYM-KONJUGATS, INSBESONDERE ZUR ELEKTROCHEMISCHEN DETEKTION DES ANALYTEN**

METHOD AND SYSTEM FOR DETERMINING THE CONCENTRATION OF AN ANALYTE/ENZYME COMPLEX OR OF AN ANALYTE/ENZYME CONJUGATE, ESPECIALLY FOR THE ELECTROCHEMICAL DETECTION OF THE ANALYTE

PROCEDE ET SYSTEME POUR DETERMINER LA CONCENTRATION D'UN COMPLEXE ANALYTE-ENZYME OU D'UN CONJUGUE ANALYTE-ENZYME, EN PARTICULIER POUR DETECTER L'ANALYTE PAR VOIE ELECTROCHIMIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **04.08.2005 DE 102005037436**

(43) Veröffentlichungstag der Anmeldung:
**16.04.2008 Patentblatt 2008/16**

(73) Patentinhaber: **Siemens Aktiengesellschaft
80333 München (DE)**

(72) Erfinder:
- **BARLAG, Heike
90403 Nürnberg (DE)**
- **MUND, Konrad
91080 Uttenreuth (DE)**
- **GUMBRECHT, Walter
91074 Herzogenaurach (DE)**

(56) Entgegenhaltungen:

**WO-A-00/62048 WO-A-01/36958
WO-A-03/043945 WO-A-2005/073705
WO-A-2005/073708 WO-A2-02/20838
US-A1- 2004 072 158 US-B1- 6 682 648**

- **GUNASINGHAM HARI ET AL: "Pulsed amperometric detection of glucose using a mediated enzyme electrode" JOURNAL OF ELECTROANALYTICAL CHEMISTRY AND INTERFACIAL ELECTROCHEMISTRY, ELSEVIER, AMSTERDAM, NL, Bd. 287, Nr. 2, 25. Juli 1990 (1990-07-25), Seiten 349-362, XP002328306 ISSN: 0022-0728**
- **BINDRA, D. S. AND WILSON, G.S.: "Pulsed Amperometric Detection of Glucose in Biological Fluids at a Surface-Modified Gold Electrode" ANALYTICAL CHEMISTRY, Bd. 61, 1989, Seiten 2566-2570, XP002409735**
- **R. THEWES ET AL.: "Sensor Array for Fully-Electronic DNA Detection on CMOS" ISSCC 2002, Nr. 21.2, 2002, XP002429975**
- **R. THEWES ET AL.: "CMOS-Sensoren für Life-Science" VDE-KONGRESS, BERLIN 2004, 2004, Seiten 81-88, XP002429976**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung bezieht sich auf ein Verfahren und ein System zur Konzentrationsbestimmung eines Analyt-Enzym-Komplex oder Analyt-Enzym-Konjugats, insbesondere zur elektrochemischen Detektion des Analyten. Bei einem solchen Verfahren wird über das Enzym ein Substrat durch hydrolytische Spaltung zu einem Reportermolekül umgesetzt, dessen Konzentration oder Konzentrationsänderung im ruhenden Elektrolyten durch ein amperometrisches Messverfahren bestimmt wird, das auf der Oxidation und Reduktion des Reportermoleküls an derselben Edelmetallelektrode basiert.

Zur Bestimmung von Antikörpern, Antigenen oder DNA in Blut, Wasser, Luft oder Lebensmitteln werden sogenannte Biosensoren eingesetzt. Sie basieren auf der spezifischen Bindung des Analyten mit einem Fängermolekül, also z.B. eines Antikörpers mit einem Antigen oder einer DNA-Sequenz mit der komplementären DNA-Fängersequenz. Der Nachweis dieses Bindungsereignisses erfolgt häufig durch optische Verfahren. Dabei werden Fluoreszenzfarbstoffe z.B. bei einer DNA-Analyse durch die PCR in den Analyten eingefügt und später an den Positionen mit verschiedenen Fängersequenzen ausgelesen. Solche optischen Systeme sind allerdings teuer und aufwändig in der Handhabung. Die erforderlichen Geräte sind empfindlich und für Feldtests nicht geeignet.

Eine geeignete Alternative sind hier elektrochemische

Biosensoren. Hier erfolgt die Detektion durch die Umsetzung einer elektrochemischen Substanz. Weitverbreitet ist die elektrochemische Detektion z.B. bei den Glucosesensoren. Die Glucose wird durch ein Redoxenzym, die Glucoseoxidase, oxidiert und gleichzeitig wird anwesender Sauerstoff reduziert. Das entstandene Wasserstoffperoxid wird dann elektrochemisch wieder oxidert und so amperometrisch die Glucosekonzentration bestimmt. In Weiterentwicklungen dieser Glucosesensoren ersetzen Mediatoren den Sauerstoff. Bei der Oxidation der Glucose durch das Redoxenzym wird gleichzeitig der Mediator, z.B. 1,1-Dimethylferrocen, reduziert. Die zur amperometrischen Bestimmung der Glucosekonzentration elektrochemische Oxidation des enzymatisch reduzierten Mediators kann hier bei deutlich niedrigeren Potenzialen erfolgen, wodurch die Messung genauer und weniger störungsanfällig wird. In diesem Zusammenhang sind zahlreiche amperometrische Pulsverfahren entwickelt worden, die nicht die Gesamtmenge an Redoxmediator bestimmten, sondern nur den vorher enzymatisch reduzierten Anteil.

Bei der elektrochemischen Detektion von Bindungsereignissen muss jedoch die absolute Menge einer redoxaktiven Substanz möglichst empfindlich gemessen werden. Der eigentliche Marker für die Anwesenheit des Analyten an einer Sensorposition ist das Enzym selbst. Bei der DNA-Analyse wird z.B. bei der PCR ein Biotinmarker an den Analyten angefügt. Auf einem Biosensor sind an den verschiedenen Sensorpositionen unterschiedliche DNA-Fängersequenzen gebunden. Der Analyt hybridisiert nur an die passenden Sequenzen und der ungebundene Analyt wird weggewaschen. Zur Erkennung dieses Bindungsereignisses wird nun an das Biotin-Markermolekül ein Streptavidin-Enzym, z.B. alkalische Phophatase, gebunden. Wird Enzymsubstrat, z.B. p-Aminophenylphosphat, zugegeben, so wird nur an den Sensorpositionen, an denen der Analyt gebunden hat, durch die hydrolytische Spaltung des Phosphats, p-Aminophenol freigesetzt.

Die Verwendung von p-Aminophenylphosphat als Substrat für die alkalische Phosphatase wird in CLIN. CHEM. 36/11, S.1941 - 1944 (1990) eingeführt, wo ein ein Bead-basierter Immunoassay beschrieben wird. Ob Analyt an die Bead-immobilisierten Antikörper gebunden hat, wird auch hier durch alkalische Phosphatase angezeigt. Die Beads werden mit p-Aminophenylphosphat inkubiert und dann die überstehende Lösung in einem Flow-Injection-Analysesystem auf p-Aminophenylphosphat untersucht. Dabei fließt diese Lösung über einen elektrochemischen Sensor, dessen Arbeitselektrode konstant bei ca. +0,1 V vs. Ag/AgCl-Referenzelektrode polarisiert ist. Die Messung im fließenden Elektrolyten bzw. Probenvolumen hat den Vorteil, dass keine nennenswerte Verarmung des p-Aminophenols vor der Elektrode statt findet. Durch den Fluss wird es ständig nachgeliefert. Ein solchen Systems ist allerdings nicht mikrosystemfähig. Der elektrochemische Sensor kann nur eine Probe zur Zeit auslesen und die benötigten Volumina sind groß.

Sofern man ein Array von Sensoren mit unterschiedlichen Fängern - seien es nun DNA-Fängersequenzen oder Antikörper -, verwenden will, so muss die elektrochemische Detektion im ruhenden Elektrolyten durchgeführt werden, damit die Sensoren nur das Signal von den direkt darauf immobilisierten Fängern detektieren. Die Anwesenheit des Enzyms wird durch einen Anstieg der p-Aminophenolkonzentration angezeigt. Einfache elektrochemische Sensoren, bei denen die Arbeitselektrode auf einem konstanten Potenzial liegt, sind hierzu nicht geeignet. Durch die ständig ablaufende Umsetzung des Enzymprodukts wird dieses verbraucht. Der Anstieg der Konzentration durch die Enzymaktivität wird also überlagert von einer Abnahme der Konzentration durch die Messung selbst.

Um dieses Problem zu umgehen wird in der US 6 682 648 B1 der Einsatz von Interdigitalelektrodenarrays vorgeschlagen. Jeder Sensor besteht aus zwei Interdigitalelektroden. Mittels eines Bipotentiostaten wird eine der Elektroden positiv und die andere negativ polarisiert. Das p-Aminophenol wird an der ersten Elektrode oxidiert und damit verbraucht. Kann es nun zur zweiten Elektrode diffundieren, so wird es dort wieder reduziert und steht der Messung an der ersten Elektrode wieder zur Verfügung.

Vorraussetzung für letzteres Redoxcyclingsystem ist jedoch, dass der Abstand zwischen diesen Elektroden sehr gering ist, damit der Transport durch Diffusion von p-Aminophenol und dem Oxidationsprodukt Chinonimin zwischen den Elektroden schnell genug statt findet, wozu auf die Veröffentlichung K. Aoki, J. Electroanal. Chem, 270 (1989), S. 35 verwiesen wird. In der bereits erwähnten US 6 682 648 B1 wird hierzu die Verwendung von Interdigital-elektroden mit Strukturdimensionen kleiner 1μm vorgeschlagen. Daraus ergibt sich, dass die Herstellung eines Biosensor-arrays mit solchen Interdigitalelektroden aufwändig und teuer ist.

Weitere Hinweise zur Messung speziell an Flüssigkeiten oder auch für biochemische Messungen werden in der DE 43 35 241 A1, in der DE 41 31 731 A1, in der DE 197 17 809 U1 und in der DE 199 17 052 A1 gegeben. Ein Verfahren zur elektrochemischen Messung des Redoxcyclings mit einer praxisgerechten Elektrodenanordnung ist im Einzelnen in der WO 01/67587 A1 beschrieben.

Ausgehend von letzterem Stand der Technik ist es Aufgabe der Erfindung, ein Verfahren bzw. System vorzuschlagen, bei denen die Bestimmung von Konzentrationen einer redoxaktiven Substanz in μM-Konzentrationen an Arrays von flächigen Elektroden mit Durchmessern ≥ 30 μm, vorzugsweise ≥ 50 μm, möglich ist. Dabei soll das System nicht konvektiv sein, also weder Elektrode noch Lösung gerührt bzw. bewegt werden und die Messfrequenz ≥ 1 Hz betragen. Daneben soll eine zugehörige Messvorrichtung geschaffen werden.

Die Aufgabe ist erfindungsgemäß bei einem Verfahren der eingangs genannten Art durch die Maßnahmen des Patentanspruches 1 gelöst. Eine zugehöriges System ist im Patent-anspruch 12 angegeben. Weiterbildungen des erfindungsgemäßen Verfahrens und des diesbezüglichen Systems sind Gegenstand der abhängigen Ansprüche.

Bei der Erfindung wird vorgeschlagen, das Potenzial der Arbeitselektrode zu pulsen,Was in anderem Zusammenhang bereits schon vorgeschlagen wurde. Darüber hinaus werden aber bei vorliegender Erfindung aber nun abwechselnd Messphasen sowie Relaxationsphasen gebildet, wobei die Messphasen-Pulslängen so gewählt werden, dass gegen Ende des Pulses der kapazitive Strom klein gegenüber dem Faraday'schen Strom ist, und wobei die Relaxationsphasen-Pulslängen so gewählt werden, dass gegen Ende des Pulses der Konzentrationsgradient relaxiert ist, so dass zu Beginn der folgenden Messphase die Konzentrationsänderung des redoxaktiven Enzymprodukts - verursacht durch den Verbrauch des Enzymprodukts durch die Messung selbst - weitestgehend rückgängig gemacht wird. Damit bildet der am Ende der Messphase gemessene Strom ein signifikantes Messsignal, was 'a priori' nicht zu erwarten ist.

Gemäß der Erfindung erfolgt die schnelle Messung der Konzentration und insbesondere der Konzentrationsänderung eines Enzymprodukts in molekularbiologischen Detektionssystemen mittels der elektrochemischen Redoxreaktion der enzymatisch gebildeten redoxaktiven Substanz bei den auf einem Chipband in Kavernen verankerten Arbeitselektroden durch eine zyklische Pulsbelastung, wobei der nach Abklingen der Umladung der Doppelschicht gemessene Strom das Messsignal bildet.

Grundlage der Erfindung ist die Erkenntnis, dass mit einem geeigneten Messverfahrens zur Bestimmung der Konzentration einer redoxaktiven Substanz vorteilhafterweise an Elektroden mit Durchmessern in der Größenordnung einiger 100 μm bis zu 1 cm gemessen werden kann. Insbesondere komplex aufgebaute Interdigitalelektroden sind nicht mehr notwendig. Nunmehr können insbesondere auch preisgünstige Transducer-Arrays verwendet werden, wie sie im Einzelnen beispielsweise in der nicht vorveröffentlichten deutschen Patentanmeldung AZ. 10 2004 004 654.9-52 der Anmelderin beschrieben werden.

Bei der zugehörigen Messvorrichtung, die nicht Teil der Erfindung ist, vereinfacht sich der Messaufbau gegenüber dem Redoxcycling dahingehend, dass kein Bipotentiostat benötigt wird. Ein einfacher Potentiostat in Kombination mit einem Pulsgenerator reicht aus.

Bei der Erfindung wird nicht - wie beim bekannten Redoxcycling - ein stationärer Zustand eingestellt, sondern eine schnelle Relaxation des Konzentrationsgradienten elektrochemisch erzwungen. Dazu wird das Potenzial der Arbeitselektrode gepulst. Es bildet sich eine Diffusionsschicht aus, deren Dicke am Ende der Messperiode einen Maximalwert erreicht, der von der Länge der Messphase abhängt. Bei einer Messung von Oxidationsströmen wird während der Relaxationsphase ein hinreichendes Reduktionspotenzial eingestellt. Die während der Messphase oxidierten und noch vor der Elektrode befindlichen Spezies werden so wieder reduziert und der Konzentrationsgradient und damit die Diffusionsschicht werden abgebaut.

Statt also wie beim Redoxcycling konstante Diffusionsschichtdicken zu etablieren, wird bei der erfindungsgemäßen "Forced Relaxation Amperometry" die Diffusionsschicht zeitlich auf- und wieder abgebaut. In beiden Fällen wird dadurch eine zumindest in ihrem Maximalwert begrenzte Diffusionsschichtdicke eingestellt. Soll eine Reduktion beobachtet werden, so muss während der Messphase das Reduktionspotenzial und während der Relaxationsphase das entsprechende Oxidationspotenzial eingestellt werden.

Weitere Einzelheiten und Vorteile der Erfindung ergeben sich aus der nachfolgenden Figurenbeschreibung von Ausführungsbeispielen anhand der Zeichnung in Verbindung mit den Patentansprüchen. Es zeigen

| | |
|---|---|
| Figur 1 bis Figur 4 | schematisch die Methodik zur Verdeutlichung der Erfindung, |
| Figur 5 | eine graphische Darstellung der forced Relaxation Amperometry mit Kennlinien für Pulslängen und zugehörigen Konzentrations- und Signalkurven, |

| | |
|---|---|
| Figur 6 | eine graphische Darstellung der Abhängigkeit der relativen Konzentration vom Abstand der Elektroden, |
| Figur 7 | eine graphische Darstellung des Stromes von der Konzentration und |
| Figur 8 | eine graphische Darstellung der Steigung in Abhängigkeit vom Potenzial während der Relaxationsphase, |
| Figur 9 | eine graphische Darstellung der Abhängigkeit der Stromdichte von der Zeit, |
| Figur 10 | eine Darstellung entsprechend Figur 5 für die Relaxationsphase, |
| Figur 11 | eine Darstellung entsprechend Figur 5 mit Vergleich von experimentellen und berechneten Werten, |
| Figur 12 | eine graphische Darstellung der Abhängigkeit der Konzentration vom Elektrodenabstand |
| Figur 13 | eine Messvorrichtung für die forced Relaxation Amperometry mit einem zugehörigen TransducerArray, |
| Figur 14/15 | ein erstes Transducer-Array für die Messvorrichtung gemäß Figur 9 in der Sicht von oben und unten, |
| Figur 16 | ein zweites, Silicium-basiertes Transducer-Array in der Draufsicht und |
| Figur 17 | den Verlauf einer typischen Messung mit einer Messstromkurve. |

In den Figuren 1 bis 4 wird zunächst die Methodik der sog. "Forced Relaxation Amperometry (FRA)" beschrieben. Dabei wird davon ausgegangen, dass die Methodik des Redox-Cyclings an sich vom Stand der Technik bekannt ist.

In der Figur 5 sind die zugehörigen Signalkurven für die anhand der Figuren 1 bis 4 definierte FRA-Methode dargestellt. Anschließend werden anhand der Figuren 6 bis 12 Betrachtungen zur Genauigkeit der neuen Messmethode wiedergegeben und ist schließlich in Figur 13 eine konkrete Messvorrichtung wiedergegeben. Dabei ist ein exemplarisches Transducer-Array, dessen Struktur in den Figuren 14 und 15 gezeigt ist, angegeben. Ein anderes Transducer-Array zeigt Figue 16. Figur 17 gibt schließlich die Verfahrensführung bei der Anwendung der Anordnung als DNA-Sensor wieder.

Im Einzelnen bedeuten in den Figuren 1 bis 4 Bezugszeichen 1 einen Träger für wenigstens eine Messelektrode 2, der ein Strompfad 3 für einen Elektronenfluss zugeordnet ist, wobei ein Analyt 4 vorhanden ist. Es ist weiterhin ein Enzym 5 vorhanden, dessen Konzentration ein Maß für die Konzentration des Analyten 4 darstellt, indem es an den Analyten 4 direkt oder indirekt gebunden ist oder vom Analyten 4 verdrängt wird.

Zur Bindung an den Träger 1 ist in Figur 3 und 4 ein Fänger 6 angedeutet. Bezugszeichen 7 kennzeichnet ein Substrat, das von dem Enzym 5 mittels hydrolytischer Spaltung umgesetzt wird, wobei ein Reportermolekül 8 entsteht, das unter solchen Bedingungen elektrochemisch reversibel umgesetzt werden kann, bei denen das Substrat 7 stabil ist.

Die Anordnung aus Träger 1 und Elektrode 2 stellt einen Sensor dar. Die Elektrode 2 ist für die Dauer einer Messphase A so polarisiert, dass das Reportermolekül 8 oxidiert bzw. reduziert wird, und für die Dauer einer Relaxationsphase B so polarisiert, dass die oxidierte bzw. reduzierte Form des Reportermoleküls 8 an der selben Elektrode 2 wieder reduziert bzw. oxidiert wird. Die wird durch die Figuren 3 und 4 verdeutlicht. Somit ist der während der Messphase A fließende Strom ein Maß für die Konzentration des vom Enzym 5 umgesetzten Substrats 7.

Vorteilhaft ist, wenn der Analyt ein Markierungselement enthält und wenn das Enzym ein Kopplungselement enthält, welches spezifisch an das Markierungselement des Analyten bindet. Das Enzym kann auch direkt an den Analyten gebunden sein. Dabei ist in bekannter Weise auf oder in der Nähe einer Elektrode ein Fängermolekül gebunden, welches spezifisch an den Analyten bindet.

In Figur 5 sind die unterschiedlichen Phasen während der Messung gekennzeichnet. Welche die Mess- und welche die Relaxationsphase ist, hängt davon ab, ob ein Oxidations- oder Reduktionsstrom gemessen werden soll. Die Pulslängen für Oxidation und Reduktion müssen nicht gleich sein, d.h. die Zeiten $t_{Ox}$ und $t_{Red}$ können sich unterscheiden. Auch die Potenziale $\varphi_{Ox}$ und $\varphi_{Red}$ müssen nicht symmetrisch zum Redoxpotenzial $\varphi_0$ der Spezies sein.

Die Darstellung der Figur 5 zeigt den möglichen Potenzialverlauf unter Verwendung von Rechteckpulsen. Dabei sind die Potenziale $\varphi_{Ox}$ bzw. $\varphi_{RED}$ und die Zeiten $\Delta t_{ox}$ bzw. $\Delta t_{RED}$ mit der Pulsform 11 in willkürlichen Einheiten aufgetragen. Die Pulsform 11 mit den Pulslängen zur Oxidation und Reduktion muss nicht symmetrisch sein. Es ist ebenso möglich, die forced Ralaxation Amperometry mit Dreiecksspannungskurven oder Sinuskurven zu realisieren.
Im Einzelnen ist in Figur 5 die Messphase mit A und die Relaxationsphase mit B bezeichnet. Neben der Potenzialkurve 11 sind weiterhin eine Kurve 12 für die Konzentration des

Enzymprodukts vor der Elektrode und zusätzlich der elektrische Strom 13 als Messsignal dargestellt. Der für den Prozess maßgebliche Stromwert liegt also jeweils am Ende des Relaxationsintervalls vor und ist ein Maß für die relaxierte Kronzentrationsänderung.

Bei den weiteren Betrachtungen wird davon ausgegangen, dass die Konzentration eines in seiner reduzierten Form vorliegenden Moleküls gemessen werden soll. In diesem Fall ist während der Messphase das Potenzial positiv bezogen auf das Redoxpotenzial der Spezies. Die höchsten Messströme werden erreicht, wenn das Potenzial so weit im Positiven liegt, dass sich ein Diffusionsgrenzstrom einstellt. Der Strom ist dann nicht von der Kinetik der Redoxreaktion, sondern nur von der Diffusion begrenzt.

Die zeitliche Entwicklung des Konzentrationsprofils der reduzierten Spezies ergibt sich aus der entsprechenden Lösung der Fickschen Gesetze für die Diffusion in den einfach unendlichen Halbraum, d.h.:

$$c(t,x) = c_\infty \cdot \mathrm{erf}\left(\frac{x}{2\sqrt{Dt}}\right), \quad s \to \infty \qquad (1)$$

Dabei bedeuten:

c: Konzentration als Funktion von Zeit und Ort
$c_\infty$: Konzentration in der Lösung ($x \to \infty$)
erf: Fehlerfunktion
D: Diffusionskoeffizient
s: Ausdehnung des Elektrolytraums vor der Elektrode

Eine weitere Erhöhung des Messsignals läßt sich durch eine Begrenzung des Elektrolytraums vor der Elektrode erreichen. Das vom Enzym gebildete Reportermolekül diffundiert nur zu einem Teil zur Elektrode. Der andere Teil diffundiert in den von der Elektrode abgewandten Elektrolytraum. Wird nun der Elektrolytraum soweit verkleinert, dass er kleiner als die Diffusionslänge während der Messphase ist, so findet noch während der Messphase ein Sättigung des Elektrolytraums mit Reportermolekül statt und jedes weitere gebildete Reportermolekül erhöht die Konzentration vor der Elektrode. Dies gilt für die Gesamtkonzentration an reduziertem und oxidiertem Reportermolekül. Da jedoch bei sehr kleinem Elektrolytraum die Durchführung des biochemischen Assays erschwert wird, ergibt sich daraus die Forderung nach einem Elektrolytraum mit variierbarer Höhe. Erst zu Beginn der Messung wird der Elektrolytraum verringert und damit die Empfindlichkeit des Sensors erhöht.

Durch die Verringerung des Elektrolytraums wird außerdem die Menge an Reportermolekül, die durch Diffusion vom Elektrolytraum über einem Sensor zum Elektrolytraum über einem zweiten Sensor gelangt, reduziert. Eine Verfälschung der Sensorsignale durch benachbarte Sensoren wird dadurch verringert und die Selektivität des Sensorarrays verbessert.

In Figur 6 sind zwei Konzentrationsprofile 21 und 22 für eine Substanz mit dem Diffusionskoeffizienten D = 3,6E-6 $cm^2/s$ gezeigt. Dies entspricht dem Diffusionskoeffizienten des Paraaminophenols (pAP), an dessen Beispiel die Funktionsweise der "Forced Relaxation Amperometry" im Weiteren demonstriert werden soll, wozu nachfolgend die chemische Umsetzung wiedergegeben ist:

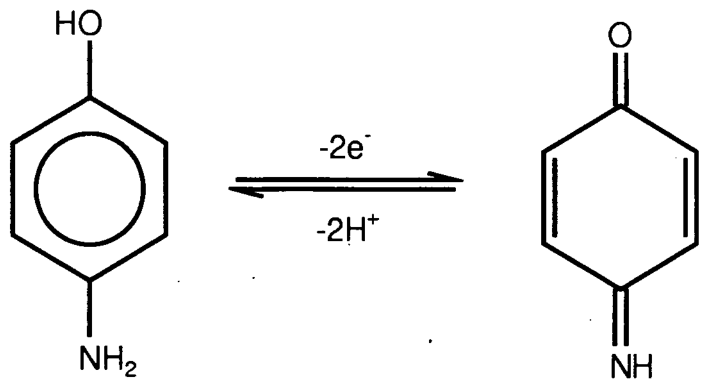


Paraaminophenol Chinonimin

Nach 0,1 s hat die Diffusionsschicht eine Dicke von ca. 25 μm. Nach 0,25 s beträgt die Ausdehnung der an pAP verarmten Schicht schon 40 μm. Je dicker diese Schicht ist, desto länger dauert die Relaxation durch Diffusion.

Die Figur 7 zeigt die Stromdichte als Funktion der pAP-Konzentration in der Lösung für verschiedene Pulsfolgen, die durch Kennlinien 31 bis 34 gekennzeichnet sind. Die Messphase beträgt immer 0,25 s, die Relaxationsphase 0,75 s. Die Strommessung erfolgt 0,24 s nach Beginn der Messphase. Das Oxidationspotenzial während der Messphase beträgt bezogen auf das Redoxpotenzial +200 mV. Variiert wurde das Potenzial während der Relaxationsphase. Es nimmt Werte zwischen -300 mV und 0 mV bezogen auf das Redoxpotenzial ein. Im Einzelnen ist:

$\varphi_{red}/\varphi_{ox}$ bei Kennlinie 31 -300 mV/200 mV,
bei Kennlinie 32 -200 mV/200 mV,

bei Kennlinie 33 -100 mV/200 mV
und bei Kennlinie 34 0 mV/200 mV.

Es ergeben sich bei diesen Parametern unterschiedliche Steigungen der Stromdichte j in ihrer funktionalen Abhängigkeit von der Konzentration pAP.

Die Steigung der Stromdichte mit der pAP-Konzentration, also die Empfindlichkeit der Messung, nimmt stetig mit negativer werdendem Potenzial während der Relaxationsphase zu. Eine Auftragung der Steigung gegen das Relaxationspotenzial entsprechend Figur 8 zeigt mit der Kennlinie 41 deutlich die vorteilhafte Wirkung der forced Relaxation Amperometry.

Während bei einem Relaxationspotenzial von 0 V vs. $\varphi_0$ die Steigung nur 462 Acm/mol beträgt, erhöht sich dieser Wert auf 864 Acm/mol bei -300 mV vs. $\varphi_0$. Diese Verdopplung beruht auf der verbesserten Winkung des Redoxcyclings, weil bei einem Relaxationspotenzial von-300 mV vs. $\varphi_0$ das Chinonimin vor der Elektrode vollständig zu Paraaminophenol (pAP) reduziert wird. Bei einem Relaxationspotenzial von 0 V vs. $\varphi_0$ wird hingegen gemäß der Nernstschen Gleichung direkt vor der Elektrode ein Konzentrationsverhältnis

Paraaminophenol:Chinonimin = 1:1

eingestellt. Das Chinonimin wird also nur teilweise wieder reduziert.

Die Steigerung der Empfindlichkeit ist nur ein, aber entscheidender Vorteil der "Forced Relaxation Amperometry". Die Konstanz des Stromsignals schon während der ersten Sekunden der Messung ist ein weiterer wesentlicher Vorteil.

Für die Bestimmung der Empfindlichkeit wurde die sich nach längerer Messzeit eingestellte Stromdichte eingesetzt. Bei der Messung von Enzymaktivitäten z.B. erfolgt die Messung aber so, dass die Lösung zunächst noch gerührt bzw. gepumpt wird. Das vom Enzym gebildete pAP wird dadurch weggespült und es stellt sich ein konstanter Grundstrom ein. Dann wird die Pumpe gestoppt und die ansteigende Konzentration während der ersten Sekunden gemessen. Typische Steigungen liegen dabei in der Größenordnung von 2 $\mu A/cm^2 s$. Führt nun die Messung selbst zu einem Absinken des Signals, so überlagern sich beide Effekte und es wird eine zu geringe Steigung des Stromes und damit Enzymaktivität gemessen. Da dieses Absinken des Stromes durch den Verbrauch der Substanz außerdem von dessen Konzentration abhängt, lässt sich dieser Effekt nicht durch Normierungen eliminieren.

Experimente mit konstanter Konzentration geben Aufschluss über die zeitliche Signalkonstanz. Die Konzentration betrug 50 $\mu M$ pAP, das Potenzial während der Messphase +200 mV. Die Dauer der Messphase Betrug 250 ms, wobei die Strommessung nach 240 ms erfolgte. Das Potenzial während der Relaxationsphase betrug im bei einem ersten Experiment 0V vs. $\varphi_0$, in einem zweiten Experiment-300 mV vs. $\varphi_0$. Die Dauer der Relaxationsphase wurde zwischen 250 ms und 4,75 s variiert.

In Figur 9 ist in einer graphischen Darstellung die Abhängigkeit der Stromdichte j von der Zeit wiedergegeben: Es ergeben sich Kennlinien 51 bis 54 für unterschiedliche Relaxationsphasendauern $\Delta t_{Red}$, und zwar im Einzelnen zwischen 0,255 und 4,755. Die Ströme fallen innerhalb der ersten 10 s der Messung stark ab. Bei einer Länge der Relaxationsphase von 0,25 s beträgt die Abnahme 14 $\mu A/cm^2$ in 10 s. Wird die Dauer der Relaxationsphase auf 4,75 s erhöht, so erniedrigt sich die Abnahme des Signals auf 9 $\mu A/cm^2$ in 10 s. Je kürzer also die Dauer der Relaxationsphase ist, desto stärker ist diese Abnahme des Signals mit der Zeit. Aber selbst bei langen Relaxationszeiten ist die Abnahme von 0,9 $\mu A/cm^2 s$ noch erheblich verglichen mit den Steigungen, die in der Größenordnung 2 $\mu A/cm^2 s$ in der Anwendung gemessen werden sollen.

Wird nun das Potenzial während der Relaxationsphase auf -300 mV heruntergesetzt, verbessert sich die Signalkonstanz signifikant. Die ergibt sich insbesondere aus Figur 6, die eine Figur 5 entsprechende Darstellung mit Kennlinien 61 bis 64 für die gleichen Parameter der Relaxationsphasen $\Delta t_{Red}$ zeigt.

Bei einer Relaxationszeit von 0,25s beträgt der Signalabfall noch 8 $\mu A/cm^2$ in 10 s. Mit einer Relaxationszeit von 0,75 s beträgt dieser Wert noch 2 $\mu A/cm^2$ in 10 s, bei 1,75 noch 1 $\mu A/cm^2$ in 10 s und für 4,75 s nur noch 0,5 $\mu A/cm^2$ in 10 s. Schon bei einer Relaxationszeit von 0,75 s, also einer Messfrequenz von 1 Hz, und einem Relaxationspotenzial von-300 mV beträgt der Signalabfall und damit auch der Fehler nur noch ca. 1 % vom erwarteten Messwert.

Die Experimente zeigen den Einfluss von Dauer und Potenzial der Relaxationsphase auf das Messsignal. Weiteren Aufschluss über die Wirkung der forced Relaxation Amperometry können Simulationsrechnungen geben.

Dabei wird zum einen die Stromdichte beim Redoxcycling berechnet zum anderen wird zum Vergleich die Stromdichte ohne Redoxcycling bestimmt.

Für die Simulationen mit Redoxcycling wurde angenommen, dass der Elektrolytraum eine Dicke von 100 $\mu$m hat. Sowohl Oxidations- als auch Reduktionspotenzial sind so gewählt, dass die Reaktion im Diffusionsgrenzstrombereich abläuft, die Ströme also maximal sind. Die Pulslängen sind 250 ms beim Oxidationspotenzial und 750 ms beim Reduktionspotenzial. Bei den Berechnungen ohne Redoxcycling waren die Parameter gleich, bis auf die Tatsache, dass während der Relaxationsphase kein Potenzial vorgegeben wird und über den Potentiostaten kein Strom fließen kann. In dieser Zeit ist das System also elektrochemisch entkoppelt.

Der Vergleich der Simulationsdaten erfolgt mit den experimentellen Ergebnissen für die entsprechenden Pulslängen und die Potenziale $\varphi_{Ox}$ = +200 mV und $\varphi_{Red}$ = -300 mV. Diese Potenzialgrenzen entsprechen am ehesten den Vorgaben für die Simulation. Die y-Achsenabschnitte der Simulationsdaten wurden den experimentellen Ergebnissen angepasst.

Die Figur 11 zeigt eine gute Übereinstimmung von Experiment und Simulation für die forced Relaxation Amperometry, wobei 71 die gemessenen Werte und 72 die gerechneten Kennlinien darstellen. Der Abfall der Stromdichte ist unter diesen Bedingungen mit 2 $\mu A/cm^2$ in 4 s gering. Ohne "Forced Relaxation Amperometry" hingegen beträgt der Stromdichteabfall während der ersten 4 s schon 12 $\mu A/cm^2$, was durch die Kennlinie 73 verdeutlicht ist Dazwischen liegen die Ergebnisse für ein Messverfahren mit Potentiostatierung während der Relaxationsphase beim Redoxpotenzial, was durch Kennlinie 74 wiedergegeben ist.

Die Verbesserung der Signalkonstanz um den Faktor 6 lässt sich direkt auf die Konzentrationsprofile zurückführen. Die folgende Abbildung zeigt die berechneten Konzentrationsprofile von pAP als Funktion des Abstandes von der Elektrode wie sie sich am Ende der 5. Relaxationsphase darstellen.

Bei der "Forced Relaxation Amperometry" wird während der Relaxationsphase das vorher gebildete Oxidationsprodukt wieder reduziert. In Folge ist die Konzentration an pAP direkt vor der Elektrode am Ende der Relaxationsphase wieder auf den ursprünglichen Wert $c_\infty$ angestiegen. Weiter von der Elektrode entfernt ist die Konzentration nur leicht erniedrigt. Ohne "Forced Relaxation Amperometry" hingegen beträgt die Konzentration vor der Elektrode unmittelbar vor der nächsten Messphase nur noch 38 %. Auch weiter entfernt von der Elektrode ist die Konzentration deutlich abgesenkt.

Letzteres ergibt sich im Einzelnen aus Figur 12 mit Kennlinien 81 und 82: Dabei entspricht das Beispiel entsprechend Kennlinie 82 für eine Messung ohne Redoxcycling in der Praxis der Messung der Konzentration einer Substanz, die zwar oxidiert werden kann, deren Oxidationsprodukte aber nicht wieder reduzierbar sind. Analoges wäre auch der Fall bei einer Substanz, die reduziert, deren Reduktionsprodukte aber nicht wieder oxidiert werden könne. Im Fall eines biochemischen Sensors könnte das z.B. Naphthol sein, das wie auch das pAP bei einer enzymatischen Reaktion freigesetzt werden kann.

Aus der Figur 13 ist die Messvorrichtung im Einzelnen ersichtlich: Außer durch ein Transducerarray 100, das anhand der Figuren 14 und 15 noch im Einzelnen beschrieben wird, wird die Messvorrichtung im Wesentlichen durch einen geeigneten Potentiostaten 105 in Kombination mit einem Pulsgenerator 106 realisiert, der optional Rechteck-, Dreieck- oder Sinuspulse liefert. Durch zwei Operationsverstärker 107 bzw. 107', von denen einer mit 'Ground'-Potenzial verbunden ist und einem definierten Messwiderstand 108 wird der Potentiostat 105 derart konzipiert, dass geeignete Potenziale bereitgestellt werden. Dabei können die Pulslänge, die Wiederholrate und die Höhe des Potenzials vorgegeben werden. Insbesondere die Pulslängen der Messphasen und die Relaxationsphasen können separat eingestellt werden und unterschiedlich lang sein. Auch die Potenziale können unterschiedlich groß sein.

Dem Transducerarray 100 sind einzelne Elektroden zugeordnet, die bestimmungsgemäß eine Referenzelektrode RE (= reference electrode), eine Gegenelektrode CE (= counter electrode) und wenigstens eine Messelektrode WE (= working electrode) realisieren. Diese Elektroden sind als Drei-Elektrodenanordnung mit dem Potentiostaten 105 verbunden. Das Signal des Potentiostaten 105 wird an eine in Figur 9 nicht im Einzelnen dargestellte Signalverarbeitungseinheit angeschlossen, mit welcher eine Auswertung unter Berücksichtigung obiger Ausführungen zur Messmethodik und Genauigkeit erfolgt. Im Allgemeinen ergibt sich der in Figur 13 als $I_{out}$ dargestellte Signalverlauf zur Auswertung.

In den Figuren 14/16 ist das Transducerarray 100 als Teil der Messvorrichtung wiedergegeben, das planar und flexibel ist sowie insbesondere kostengünstig herzustellen ist. Wesentlich ist dabei, dass nunmehr mit der forced Relaxation Amperometry mit einem vereinfachten Transducer-Array 100 gemessen werden kann. Die Figuren 14 und 15 zeigen die Ober- und Unterseite des Transducerarrays 100 - bestehend aus einem Metallsubstrat 101 und einer Isolatorschicht 102. Auf der Oberseite sind beispielsweise kreisförmige Vertiefungen $103_i$, die als Kavitäten bezeichnet werden, dargestellt. Die Kavitäten $103_i$ entstehen durch die Strukturierung des Isolators 102. Auf dem Grund der Vertiefungen $103_i$ liegt die Oberseite des Metallsubstrats frei und bildet einen Messpunkt, sofern ein Analyt aufgebracht ist.

Die Darstellung der Rückseite zeigt durch Striche eine Strukturierung und damit eine Auftrennung des Metallsubstrats 101 in voneinander isolierte Teile. Jede Metallinsel korrespondiert mit einer Vertiefung $103_i$ auf der Vorderseite. Durch Punkte sind die möglichen Kontaktstellen für eine sog. Nadelkarte zur vereinfachten elektrischen Kontaktierung

der Metallflächen angedeutet. Wesentlich ist dabei, dass mehrere Metallinseln, vorzugsweise drei, mit einem Analyten einen Sensor definieren und mit den zugehörigen Elektroden, die eine Messelektrode WE, eine Gegenelektrode GE und eine Referenzelektrode RE bilden, zur Durchführung von elektrochemischen Messungen geeignet sind.

In anderer Ausbildung können aber auch Transducer-Arrays in CMOS-Technologie eingesetzt werden, was anhand Figur 16 beschrieben wird:

In Figur 16 sind auf der sensitiven Fläche des Sensors bzw. Trägers 1 eine Vielzahl von Mikrokavitäten 200 für die Durchführung von biochemischen Analysen angeordnet. Es sind mxn Elemente zeilen- und spaltenförmig als Transducer-Array 200 zeilen- und spaltenförmig angeordnet. Wesentlich ist dabei, dass biochemische Reaktionen bzw. Messungen gleichzeitig in den einzelnen Kavitäten erfolgen können, ohne dass bei Zugabe von Substanzen ein Übersprechen der Reaktionen aus einer ersten Kavität in eine zweite Kavität erfolgen kann.

In Figur 16 sind auf dem Träger 1 mit sensitiver Oberfläche bzw. den einzelnen sensitiven Elementen diskrete elektrische Kontaktierungen angebracht. Die Kontaktierungen bilden Eingänge für den elektrischen Messkreis. Beispielsweise sind zwei Versorgungs-Spannungseingänge $V_{dd}$, $V_{ss}$, ein Eingang GND für Massepotential, ein Eingang für ein Clock-Signal, ein Eingang $V_{in}$ für eine Steuerspannung und ein Eingang für ein Reset-Signal vorhanden. Weiterhin sind auf dem Chip 1 ein Multiplexer 210, ein "Gray counter & decoder" 215 und ein Verstärker 220 mittels Standard-Siliziumtechnik integriert. Das Messsignal wird am Ausgang 'out' erfasst, wobei bei einer Arrayanordnung mit der Vielzahl von Kavitäten als mxn Einzelsensoren ein Multiplexsignal erhalten wird, das beispielsweise mit einer Frequenz von 10 kHz ausgelesen wird.

Das auf einer einzigen Leitung 'out' ausgegebene Multiplexsignal besteht aus einem Muster von diskreten Spannungswerten, aus dem mittels eines De-Multiplexers in einem Auswertegerät die Einzelsensor-Signale gewonnen werden.

In Figur 16 wird also in einer alternativen Messanordnung statt des freitragenden und flexiblen Transducer-Arrays entsprechend den Figuren 13 bis 15 ein Transducer-Array in Dünnfilmtechnologie auf einem starren Substrat verwendet. Dabei sind flächige Elektroden vorhanden, die eine Ausdehnung haben, die größer als die Diffusionslänge ist. Eine typische Diffusionslänge beträgt für das oben angegebene Beispiel 25 $\mu$m, so dass die flächigen Elektroden eine Ausdehnung $\geq$ 30 $\mu$m, vorzugsweise $\geq$ 50 $\mu$m, haben.

Das starre Substrat ist insbesondere Silizium, das vorzugsweise mit einer Isolatorschicht versehen ist.

Auch mit derartigen Transducern, welche den Zugang zur CMOS-Technologie bei der Signalverarbeitung ermöglichen, ist das erfindungsgemäße Redoxcycling durchführbar.

Mit einer Anordnung gemäß Figur 13 und einem Transducer-Array 100 bzw. 200 alternativ gemäß Figur 14/15 oder gemäß Figur 16 wird beispielhaft ein biochemischer Sensor zur DNA-Analyse realisiert: Beispielhaft wird das anhand der Figuren 14/15 beschriebene Transducer-Array 100 aus einer Metallschicht und einer damit verbundenen Isolatorschicht mit Kavitäten $3_i$ eingesetzt. Der Durchmesser der Kavitäten $3_i$ beträgt 0,8 mm, die Tiefe 90 $\mu$m und der Abstand zwischen zwei benachbarten Messpunkten 1mm. Die Elektrodenoberflächen sind mit einer 2,3 $\mu$m dicken Goldschicht bedeckt.

Bei der so beschriebenen Vorrichtung kann die Höhe des Elektrolytraums über dem Transducer-Array während des Assay variiert werden. Dabei kann der Elektrolytraum über dem Transducer-Array durch ein flexibles Material begrenzt werden, wobei das Material durch eine von oben wirkende Kraft in Richtung Array gedrückt werden kann. Somit wird der Elektrolytraum soweit verkleinert, dass der Transport von Transportermolekülen zwischen den Sensoren des Arrays unterbunden wird.

Insgesamt besteht für obigen Anwendungszweck die Sensoranordnung also aus mindestens drei, vorteilhafterweise aber auch aus vier Elektroden. Eine der Elektroden ist dann als Referenzelektrode mit einer Silber/Silberchlorid-(Ag/AgCl)-Schicht belegt, eine andere Elektrode wird als Gegenelektrode CE verwendet und die zwei weiteren Elektroden dienen als Messelektroden WE.

Auf einer der Messelektroden wird eine synthetische Oligonukleotidsequenz der Länge 25 mittels einer endständigen Thiolgruppe an der Goldoberfläche als Positivprobe verankert. Die zweite Messelektrode bleibt als Negativprobe frei. Dann wurden beide Oberflächen mit einer Lösung von 1 mg Rinderserumalbumin pro ml 15 Minuten inkubiert und anschließend das Sensorarray in einen 100 $\mu$m tiefen Durchflusskanal eingesetzt. Zunächst werden 10 $\mu$l einer 10 pM biotinilierten Zielsequenz innerhalb von ca. 5 Minuten über die Elektroden gepumpt. Dann wird nach einem Waschschritt eine Lösung von Streptavidin markierter alkalischer Phosphatase darüber gegeben. Das Waschen erfolgt mit einer Pufferlösung von 100 mM Tris(hydroxymethyl)aminomethan titriert auf pH 8 mit Salzsäure und 130 mM NaCl. Nach abermaligem Waschen wird eine 2 mM Lösung des Enzymsubstrats Paraaminophenylphosphat (pAPP) in der Pufferlösung über das Sensorarray gepumpt. Bei Anwesenheit des Enzyms alkalische Phosphatase wird das Enzymsubstrat pAPP zu Paraaminophenol (pAP) umgesetzt.

Zur Messung sind Referenzelektrode RE, Gegenelektrode CE und jeweils eine der beiden Messelektroden WE sind jeweils in einer Drei-Elektro-denanordnung an einen Potentiostaten angeschlossen. Die Messung erfolgt mittels

"Forced Relaxation Amperometry". Während der Messphase wird das vom Enzym gebildete Paraaminophenol zu Chinonimin oxidiert. Das Oxidationspotenzial $\varphi_{Ox}$ beträgt +200 mV vs. $\varphi_0$. In der Relaxationsphase wird das gebildete Chinonimin wieder zu Paraaminophenol reduziert und zwar bei $\varphi_{Red}$ = -200 mV. Die Pulslänge der Messphase beträgt 250 ms, die der Relaxationsphase 750 ms. Die Strommessung erfolgt 240 ms nach Beginn der Messphase.

Zu Beginn der Messung ist die Positivprobe, also die Elektrode mit der Fängersequenz angeschlossen. Die Lösung mit dem Enzymsubstrat fließt - durch eine Pumpe gefördert - zunächst über die Negativprobe und dann über die Positivprobe. Durch die Fließbewegung wird von dem Enzym gebildetes pAP von den Elektroden weggespült, so dass bei eingeschalteter Pumpe der Strom konstant und gering ist. Wird nun die Pumpe gestoppt steigt die pAP-Konzentration durch die Enzymaktivität mit der Zeit an. In der Messung zeigt sich dies durch einen starken Anstieg des Stromsignals mit 20 nA/s. Wird die Pumpe wieder eingeschaltet, so sinkt das Signal wieder auf den ursprünglichen Wert. Dieser Vorgang kann beliebig oft wiederholt werden.

Die Figur 17 zeigt den zeitlichen Stromverlauf bei Pumpe "on"/"stopp" an der beschriebenen Sensoranordnung mit positiver und negativer Probe: Die Kennlinie 121 zeigt den Verlauf des Pumpenstroms. Es ergibt sich für das Experiment ein spezifischer Verlauf mit einzelnen Peaks, wobei Parameter die Aktivierung der Pumpe einerseits ("stopp"/"on") und das Umschalten der Messelektroden andererseits ist. Der interessierende Messbereich ist jeweils strichliert unterlegt. Bei t = 400 s wurde auf die Negativprobe umgeschaltet. Hier sinkt der Strom beim Stoppen der Pumpe zunächst, bleibt dann kurze Zeit konstant und steigt dann langsam an. Dieser Anstieg wird durch die Diffusion von pAP von der positiven zur negativen Probe hin verursacht. Bei Pumpe "on" kommt ein Peakstrom hinzu, da der Elektrolyt zunächst von der positiven zur negativen Probe fließt und damit eine erhöhte pAP-Konzentration zur benachbarten Elektrode transportiert. Insgesamt ist die Diskriminierung von positiver und negativer Probe sehr gut.

## Patentansprüche

**1.** Verfahren zur elektrochemischen Detektion eines Analyten, mit folgenden Maßnahmen:

- Es wird ein Enzym verwendet, dessen Konzentration ein Maß für die Konzentration des Analyten darstellt, indem es an den Analyten direkt oder indirekt gebunden ist oder vom Analyten verdrängt wird;
- es wird ein Substrat verwendet, das von dem Enzym mittels hydrolytischer Spaltung umgesetzt wird, wobei ein Reportermolekül entsteht, das unter Bedingungen elektrochemisch reversibel umgesetzt werden kann, bei denen das Substrat stabil ist;
- es wird ein Sensor mit wenigstens einer Elektrode verwendet, wobei die Elektrode für die Dauer einer Messphase (A) so polarisiert wird, dass das Reportermolekül oxidiert bzw. reduziert wird und wobei die Elektrode für die Dauer einer Relaxationsphase (B) so polarisiert wird, dass die oxidierte bzw. reduzierte Form des Reporter-moleküls an derselben Elektrode wieder reduziert bzw. oxidiert wird;
- der während der Messphase (A) fließende Strom wird als Maß für die Konzentration des vom Enzym umgesetzten Substrats erfasst.

**2.** Verfahren nach Anspruch 1, **dadurch gekennzeichnet, dass** ein Analyt mit einem Markierungselement verwendet wird.

**3.** Verfahren nach Anspruch 1, **dadurch gekennzeichnet, dass** ein Enzym mit einem Kopplungselement verwendet wird, welches spezifisch an das Markierungselement des Analyten bindet.

**4.** Verfahren nach einem der Ansprüche 1 bis 3, **dadurch gekennzeichnet, dass** das Enzym an den Analyten gebunden ist.

**5.** Verfahren nach Anspruch 3 oder 4, **dadurch gekennzeichnet, dass** auf oder in der Nähe einer Elektrode ein Fängermolekül immobilisiert wird, wobei das Fängermolekül spezifisch an den Analyten bindet.

**6.** Verfahren nach einem der vorhergehenden Ansprüche, **dadurch gekennzeichnet, dass** gegen Ende der Messphase (A) der kapazitive Strom klein gegenüber dem Faraday'schen Strom ist.

**7.** Verfahren nach Anspruch 6, **dadurch gekennzeichnet, dass** gegen Ende der Relaxationsphase (B) die während der Messphase (A) oxidierten Reportermoleküle weitestgehend wieder reduziert sind.

**8.** Verfahren nach Anspruch 6, **dadurch gekennzeichnet, dass** gegen Ende der Relaxationsphase (B) die während der Messphase (A) reduzierten Reportermoleküle weitestgehend wieder oxidiert sind.

**9.** Verfahren nach Anspruch 8, **dadurch gekennzeichnet, dass** die Potenziale derart gewählt werden, dass die Reduktions- und Oxidationsreaktionen des Reportermoleküls im Diffusionsgrenzstrombereich ablaufen.

**10.** Verfahren nach Anspruch 8, **dadurch gekennzeichnet, dass** die Wiederholrate für die Messphase (A) wenigstens 0,5 Hz beträgt.

**11.** Verfahren nach einem der vorhergehenden Ansprüche, **dadurch gekennzeichnet, dass** die Reduktion bzw. Oxidation des Reportermoleküls während der Relaxationsphase (B) vollständig erfolgt.

**12.** System zur elektrochemischen Detektion eines Analyten gemäß dem Verfahren nach Anspruch 1, mit

- einem Enzym (5), dessen Konzentration ein Maß für die Konzentration des Analyten (4) darstellt,
- einem Substrat (7), das von dem Enzym (5) mittels hydrolytischer Spaltung umgesetzt wird,
- einem Reportermolekül (8, 8'), das unter Bedingungen, bei denen das Substrat (7) stabil ist, elektrochemisch reversibel umgesetzt werden kann und
- einem Sensor (1) mit wenigstens einer Elektrode(2), die derart ausgestaltet ist, dass sie für die Dauer einer Messphase (A) so polarisierbar ist, dass das Reportermolekül (8, 8') oxidiert bzw. reduziert wird und für die Dauer einer Relaxationsphase (B) so polarisierbar ist, dass die oxidierte bzw. reduzierte Form des Reportermoleküls an der selben Elektrode (2) wieder reduziert bzw. oxidiert wird, wobei der während der Messphase (A) fließende Strom ein Maß für die Konzentration des vom Enzym (5) umgesetzten Substrats ist.

**13.** System nach Anspruch 12, **dadurch gekennzeichnet, dass** das Enzym(5) ein Kopplungselement enthält, welches spezifisch an ein Markierungselement des Analyten (5) bindbar ist.

**14.** System nach Anspruch 12, **dadurch gekennzeichnet, dass** das Enzym (5) an den Analyten (4) bindbar ist.

**15.** System nach Anspruch 12, **dadurch gekennzeichnet, dass** auf oder in der Nähe einer Elektrode ein Fängermolekül (4) gebunden ist, welches spezifisch an den Analyten (4) bindbar ist.

## Claims

**1.** Method for the electrochemical detection of an analyte, having the following measures:

- an enzyme is used whose concentration represents a measure of the concentration of the analyte, by its being bound directly or indirectly to the analyte or displaced by the analyte;
- a substrate is used which is converted by the enzyme by means of hydrolytic cleavage, a reporter molecule being formed which can be electrochemically converted reversibly under conditions in which the substrate is stable;
- a sensor having at least one electrode is used, the electrode being polarized for the duration of a measurement phase (A) so that the reporter molecule is oxidized or reduced, and the electrode being polarized for the duration of a relaxation phase (B) so that the oxidized or reduced form of the reporter molecule is respectively reduced or oxidized again at the same electrode;
- the current flowing during the measurement phase (A) is recorded as a measure of the concentration of the substrate converted by the enzyme.

**2.** Method according to Claim 1, **characterized in that** an analyte with a marking element is used.

**3.** Method according to Claim 1, **characterized in that** an enzyme with a coupling element, which binds specifically to the marking element of the analyte, is used.

**4.** Method according to one of Claims 1 to 3, **characterized in that** the enzyme is bound to the analyte.

**5.** Method according to Claim 3 or 4, **characterized in that** a capture molecule is immobilized on or in the vicinity of an electrode, the capture molecule binding specifically to the analyte.

**6.** Method according to one of the preceding claims, **characterized in that** the capacitive current is toward the end of the measurement phase (A) small compared with the Faraday current.

**7.** Method according to Claim 6, **characterized in that** the reporter molecules oxidized during the measurement phase (A) are substantially reduced again toward the end of the relaxation phase (B).

**8.** Method according to Claim 6, **characterized in that** the reporter molecules reduced during the measurement phase (A) are substantially oxidized again toward the end of the relaxation phase (B).

**9.** Method according to Claim 8, **characterized in that** the potentials are selected so that the reduction and oxidation reactions of the reporter molecule take place in the diffusion limit current range.

**10.** Method according to Claim 8, **characterized in that** the repetition rate for the measurement phase (A) is at least 0.5 Hz.

**11.** Method according to one of the preceding claims, **characterized in that** the reduction or oxidation of the reporter molecule during the relaxation phase (B) takes place completely.

**12.** System for the electrochemical detection of an analyte according to the method according to Claim 1, having

- an enzyme (5) whose concentration represents a measure of the concentration of the analyte (4),
- a substrate (7) which is converted by the enzyme (5) by means of hydrolytic cleavage,
- a reporter molecule (8, 8') which can be electrochemically converted reversibly under conditions in which the substrate (7) is stable, and
- a sensor (1) having at least one electrode (2), which is designed in such a way that it can be polarized for the duration of a measurement phase (A) so that the reporter molecule (8, 8') is oxidized or reduced and can be polarized for the duration of a relaxation phase (B) so that the oxidized or reduced form of the reporter molecule is respectively reduced or oxidized again at the same electrode (2), the current flowing during the measurement phase (A) being a measure of the concentration of the substrate (5) converted by the enzyme.

**13.** System according to Claim 12, **characterized in that** the enzyme (5) contains a coupling element which can be bound specifically to the marking element of the analyte (5).

**14.** System according to Claim 12, **characterized in that** the enzyme (5) can be bound to the analyte (4).

**15.** System according to Claim 12, **characterized in that** a capture molecule (4), which can be bound specifically to the analyte (4), is bound on or in the vicinity of an electrode.

## Revendications

**1.** Procédé de détection électrochimique d'un analyte, comprenant les mesures suivantes :

- on utilise un enzyme dont la concentration représente une mesure de la concentration de l'analyte, en ce qu'il est lié directement ou indirectement à l'analyte ou en ce qu'il est déplacé par l'analyte ;
- on utilise un substrat qui est transformé par l'enzyme au moyen d'une décomposition hydrolytique, avec une création d'une molécule reporter qui peut réagir d'une manière réversible électrochimiquement dans des conditions dans lesquelles le substrat est stable ;
- on utilise un capteur ayant au moins une électrode, l'électrode étant polarisée pour la durée d'une phase (A) de mesure de manière à oxyder ou à réduire la molécule reporter et l'électrode étant polarisée pour la durée d'une phase (B) de relaxation de manière à réduire ou à oxyder à nouveau la forme oxydée ou réduite la molécule reporter sur la même électrode ;
- on détecte le courant électrique passant pendant la phase (A) de mesure comme mesure de la concentration du substrat transformé par l'enzyme.

**2.** Procédé suivant la revendication 1, **caractérisé en ce que** l'on utilise un analyte ayant un élément de marquage.

**3.** Procédé suivant la revendication 1, **caractérisé en ce que** l'on utilise un enzyme ayant un élément de couplage, qui se fixe spécifiquement à l'élément de marquage de l'analyte.

**4.** Procédé suivant l'une des revendications 1 à 3, **caractérisé en ce que** l'enzyme est fixé à l'analyte.

**5.** Procédé suivant la revendication 3 ou 4, **caractérisé en ce que** sur ou au voisinage d'une électrode est immobilisée une molécule de capture, la molécule de capture se fixant spécifiquement à l'analyte.

**6.** Procédé suivant l'une des revendications précédentes, **caractérisé en ce que** vers la fin de la phase (A) de mesure, le courant capacitif est plus petit que le courant de Faraday.

**7.** Procédé suivant la revendication 6, **caractérisé en ce que** vers la fin de la phase (B) de relaxation? les molécules reporters oxydées pendant la phase (A) de mesure sont, dans une grande mesure, réduites à nouveau.

**8.** Procédé suivant la revendication 6, **caractérisé en ce que** vers la fin de la phase (B) de relaxation les molécules reporters réduites pendant la phase (A) de mesure sont, dans une grande mesure, oxydées à nouveau.

**9.** Procédé suivant la revendication 8, **caractérisé en ce que** l'on choisit les potentiels de manière à ce que les réactions de réduction et d'oxydation de la molécule reporter se déroulent dans la plage de courant limite de diffusion.

**10.** Procédé suivant la revendication 8, **caractérisé en ce que** le taux de répétition de la phase (A) de mesure est d'au moins 0,5 Hz.

**11.** Procédé suivant l'une des revendications précédentes, **caractérisé en ce que** la réduction ou l'oxydation de la molécule reporter s'effectue complètement pendant la phase (B) de relaxation.

**12.** Système de détection électrochimique d'un analyte suivant le procédé de la revendication 1, comprenant

- un enzyme (15) dont la concentration représente une mesure de la concentration de l'analyte (4),
- un substrat (7) qui est transformé par l'enzyme (5) au moyen d'une décomposition hydrolytique,
- une molécule (8, 8') reporter, qui peut réagir d'une manière réversible électrochimiquement dans les conditions dans lesquelles le substrat (7) est stable et
- un capteur (1) ayant au moins une électrode (2) qui est telle qu'elle peut être polarisée pendant la durée d'une phase (A) de mesure de manière à oxyder ou à réduire la molécule (8, 8') reporter et peut être polarisée pendant la durée d'une phase (B) de relaxation de manière à réduire ou à oxyder à nouveau la forme oxydée ou réduite de la molécule reporter sur la même électrode (2), le courant électrique passant pendant la phase (A) de mesure représentant une mesure de la concentration du substrat transformée par l'enzyme (5).

**13.** Système suivant la revendication 12, **caractérisé en ce que** l'enzyme (5) contient un élément de couplage, qui peut se fixer spécifiquement à un élément de marquage de l'analyte (5).

**14.** Système suivant la revendication 12, **caractérisé en ce que** l'enzyme (5) peut être fixé à l'analyte (4).

**15.** Système suivant la revendication 12, **caractérisé en ce que** sur ou à proximité d'une électrode est fixée une molécule (4) de capture, qui peut être fixée spécifiquement à l'analyte (4).

FIG 1

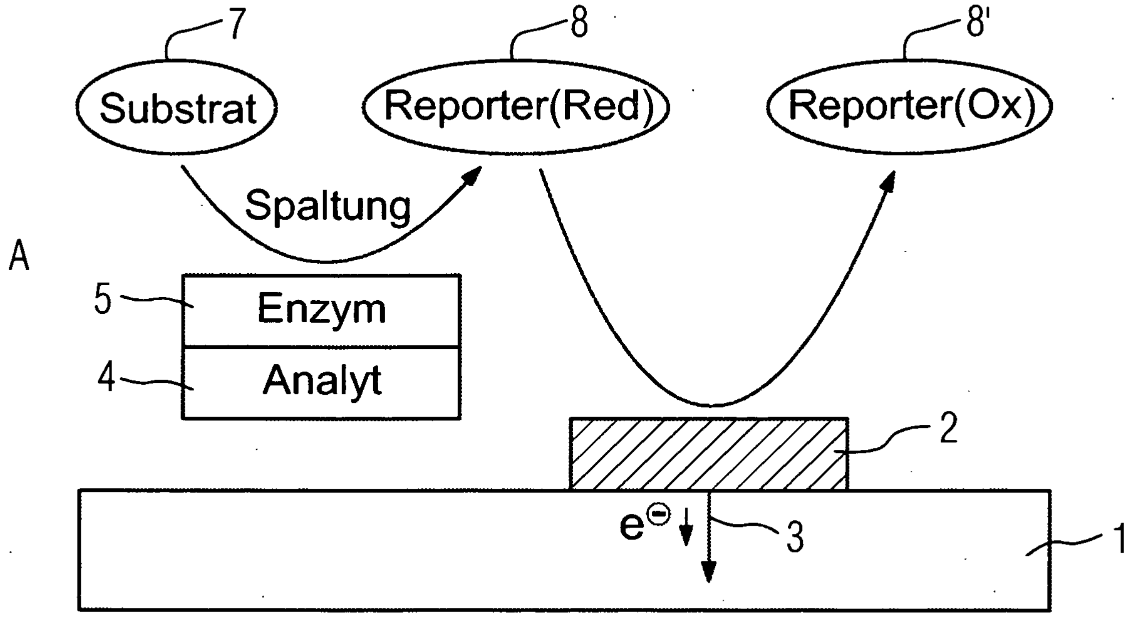
7
8
8'
Substrat
Reporter(Red)
Reporter(Ox)
Spaltung
A
5
Enzym
4
Analyt
2
e⊖
3
1

FIG 2

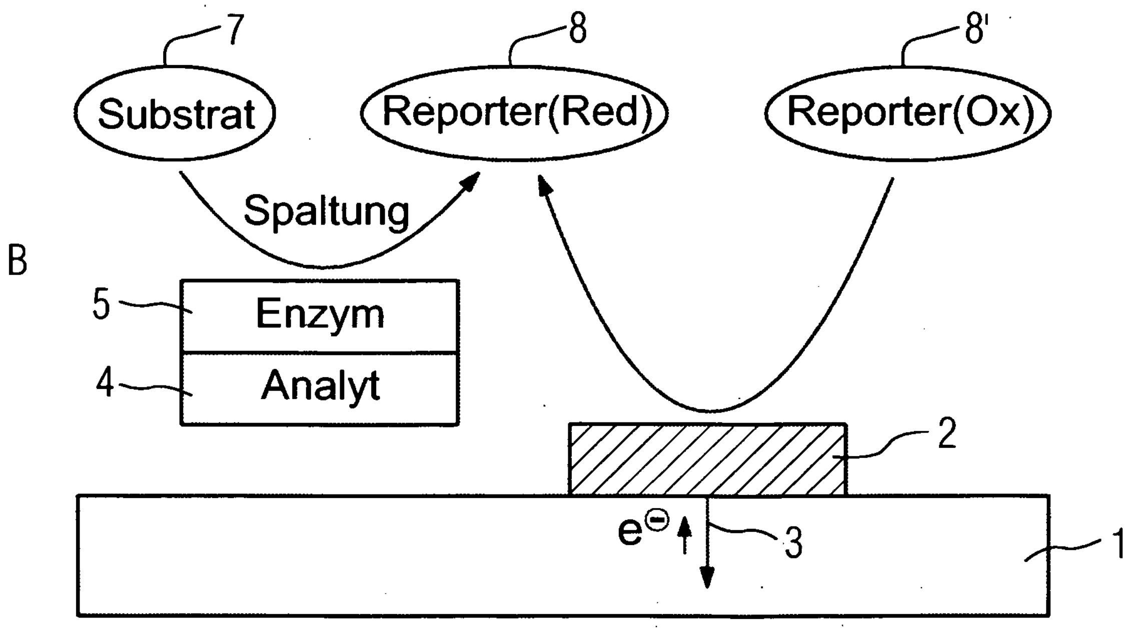
7
8
8'
Substrat
Reporter(Red)
Reporter(Ox)
Spaltung
B
5
Enzym
4
Analyt
2
e⊖
3
1

FIG 3

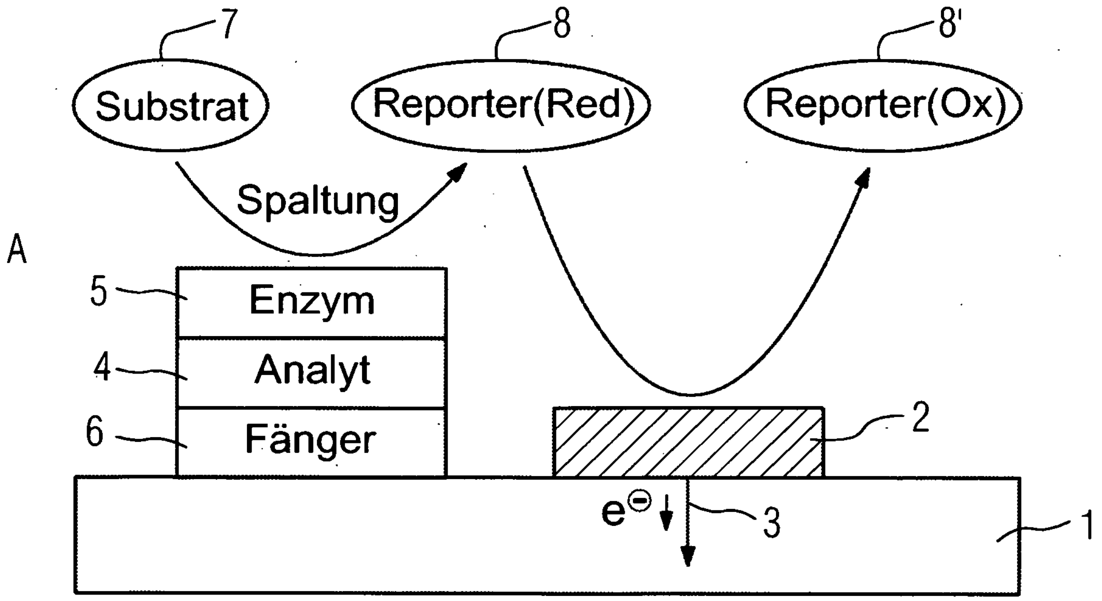
7
8
8'
Substrat
Reporter(Red)
Reporter(Ox)
Spaltung
A
5
Enzym
4
Analyt
6
Fänger
2
e⊖
3
1

FIG 4

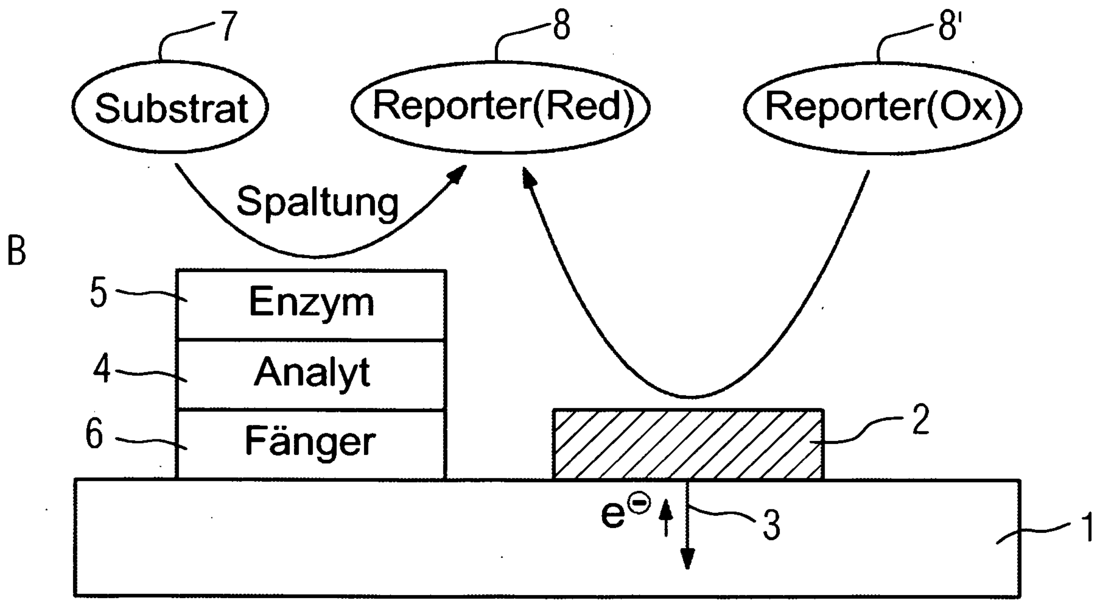
7
8
8'
Substrat
Reporter(Red)
Reporter(Ox)
Spaltung
B
5
Enzym
4
Analyt
6
Fänger
2
e⊖
3
1

FIG 5

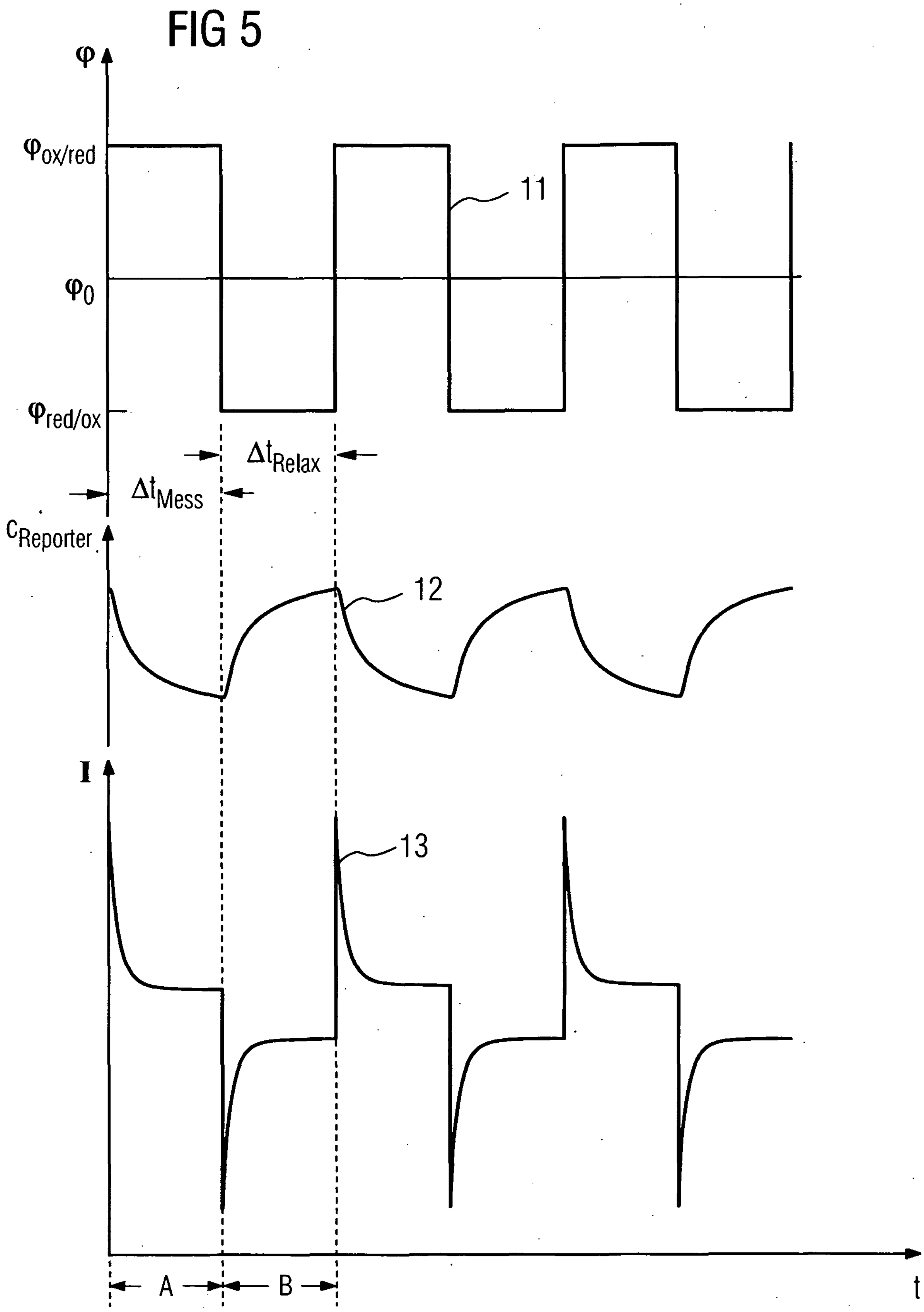

FIG 6

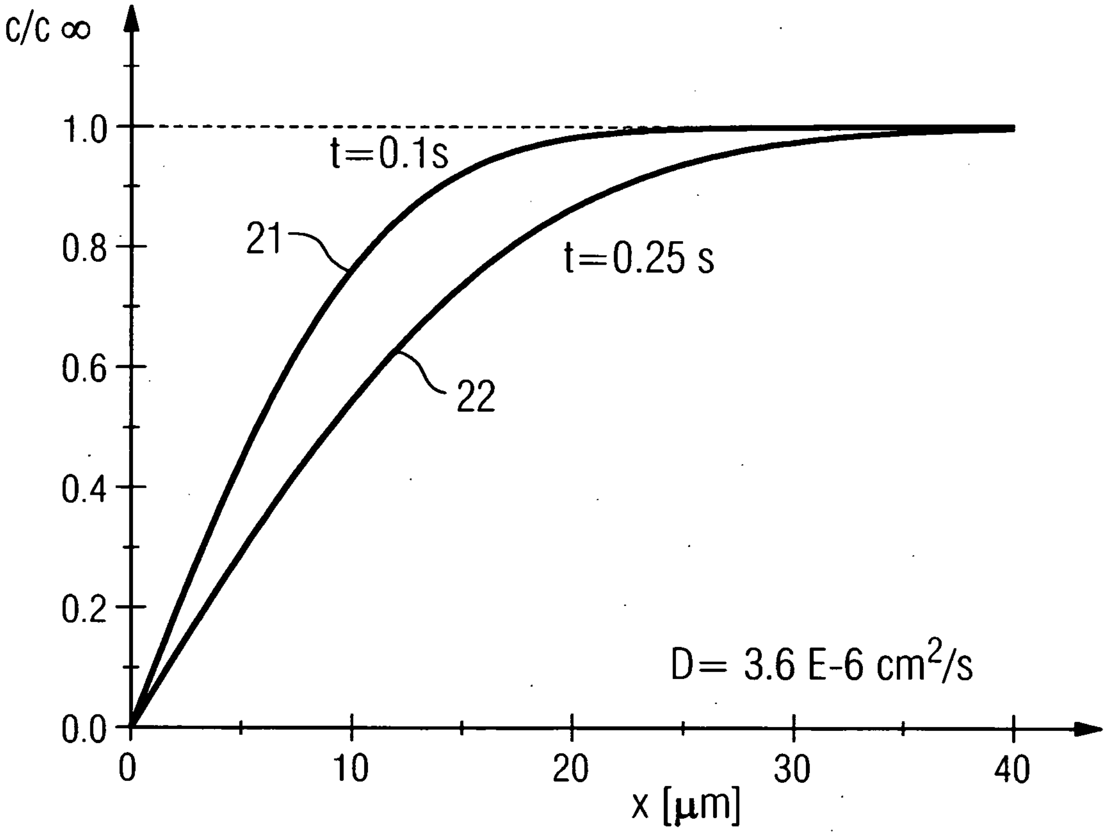

FIG 7

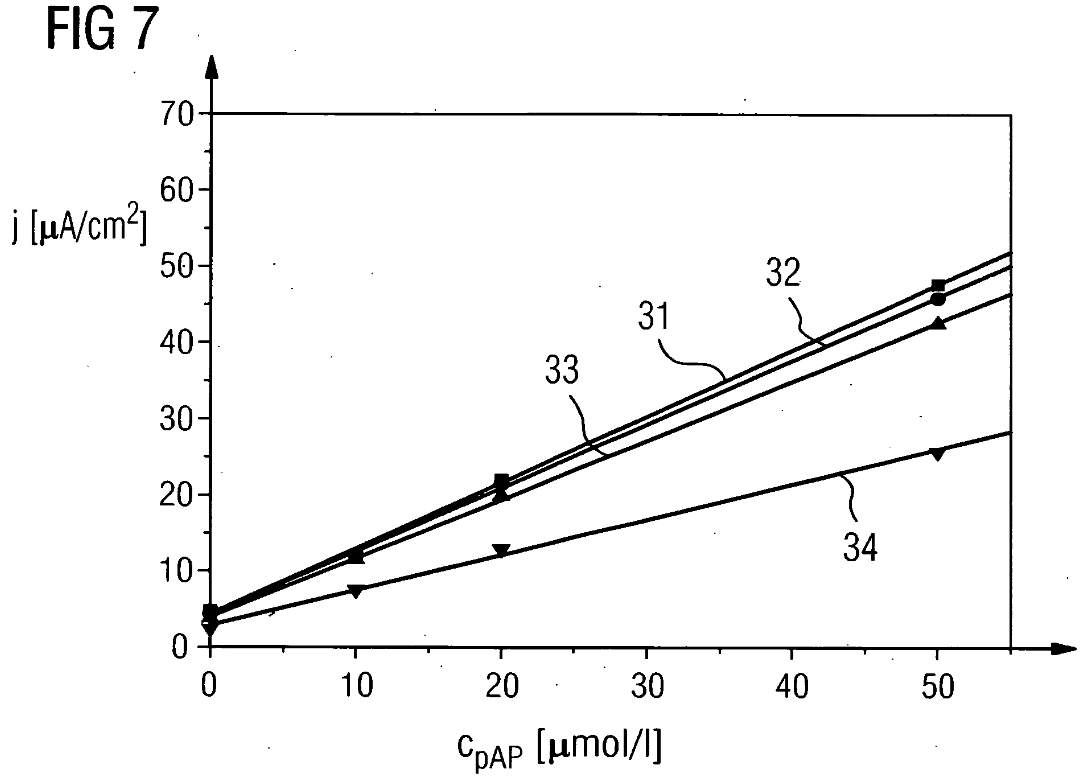
j [µA/cm2]
70
60
50
40
30
20
10
0
0
10
20
30
40
50
cpAP [µmol/l]
31
32
33
34

FIG 8

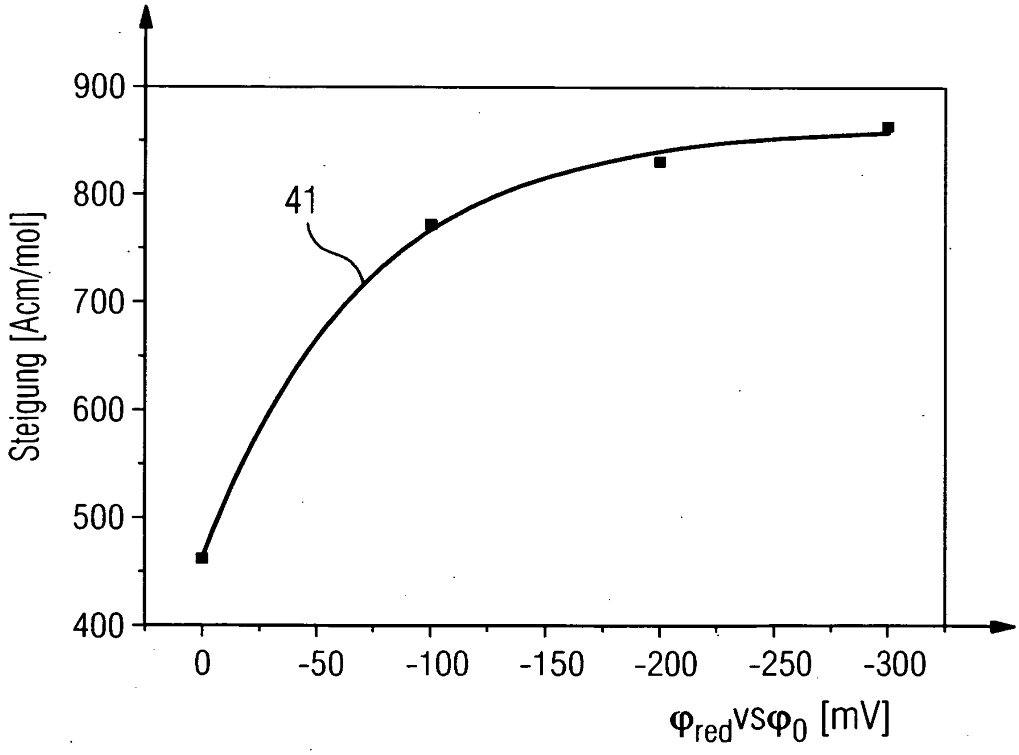
Steigung [Acm/mol]
900
800
700
600
500
400
0
-50
-100
-150
-200
-250
-300
φred vs φ0 [mV]
41

FIG 9

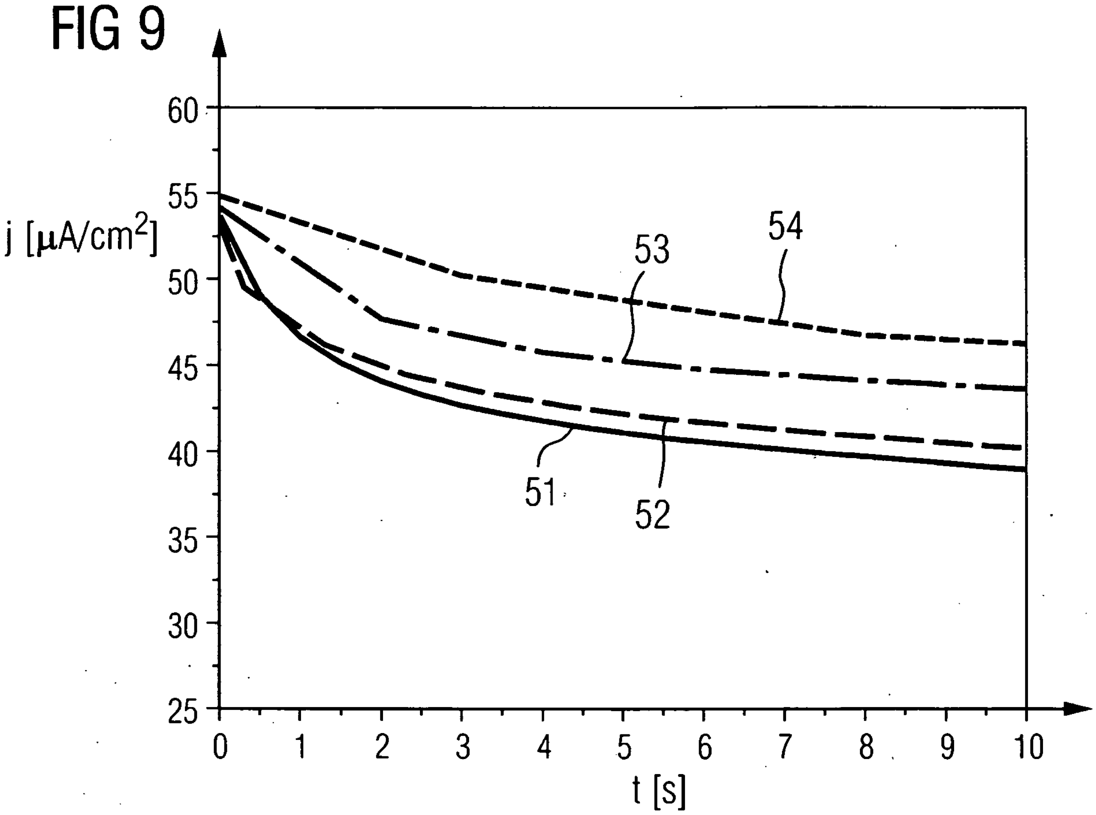
60
55
50
45
40
35
30
25
j [μA/cm2]
0
1
2
3
4
5
6
7
8
9
10
t [s]
51
52
53
54

FIG 10

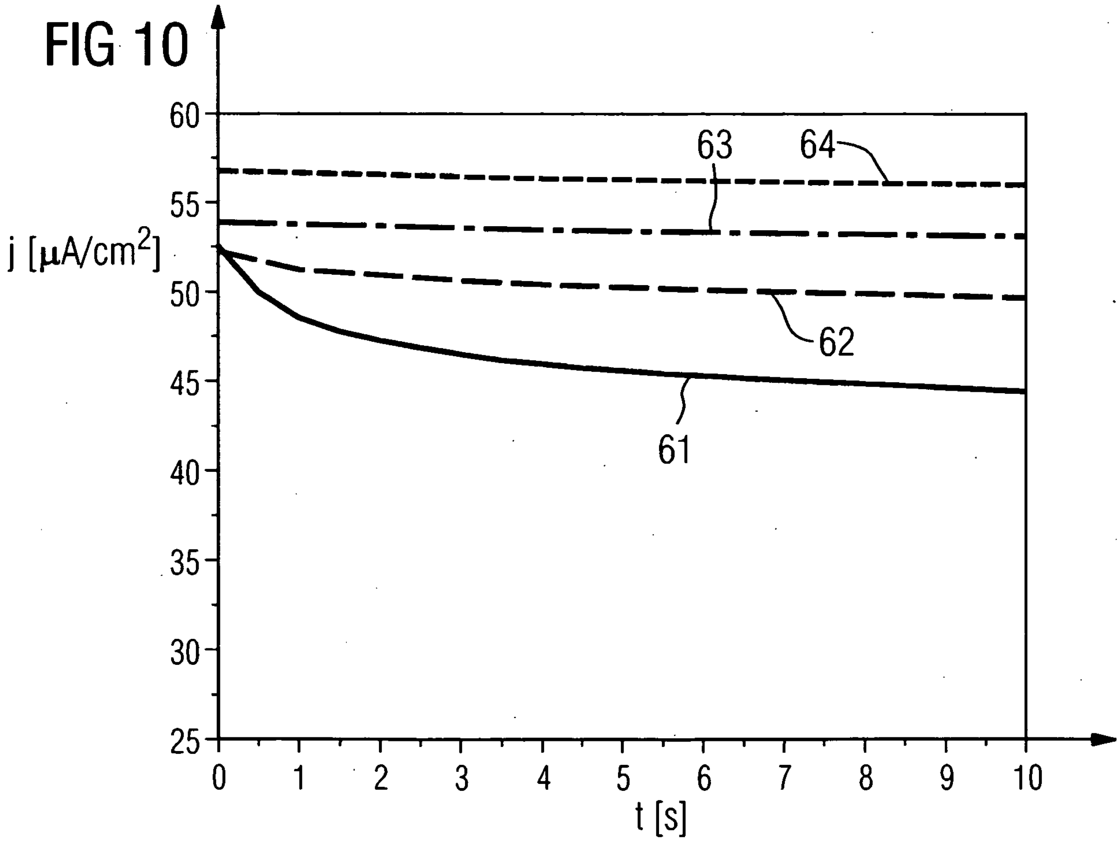
60
55
50
45
40
35
30
25
j [μA/cm2]
0
1
2
3
4
5
6
7
8
9
10
t [s]
61
62
63
64

FIG 11

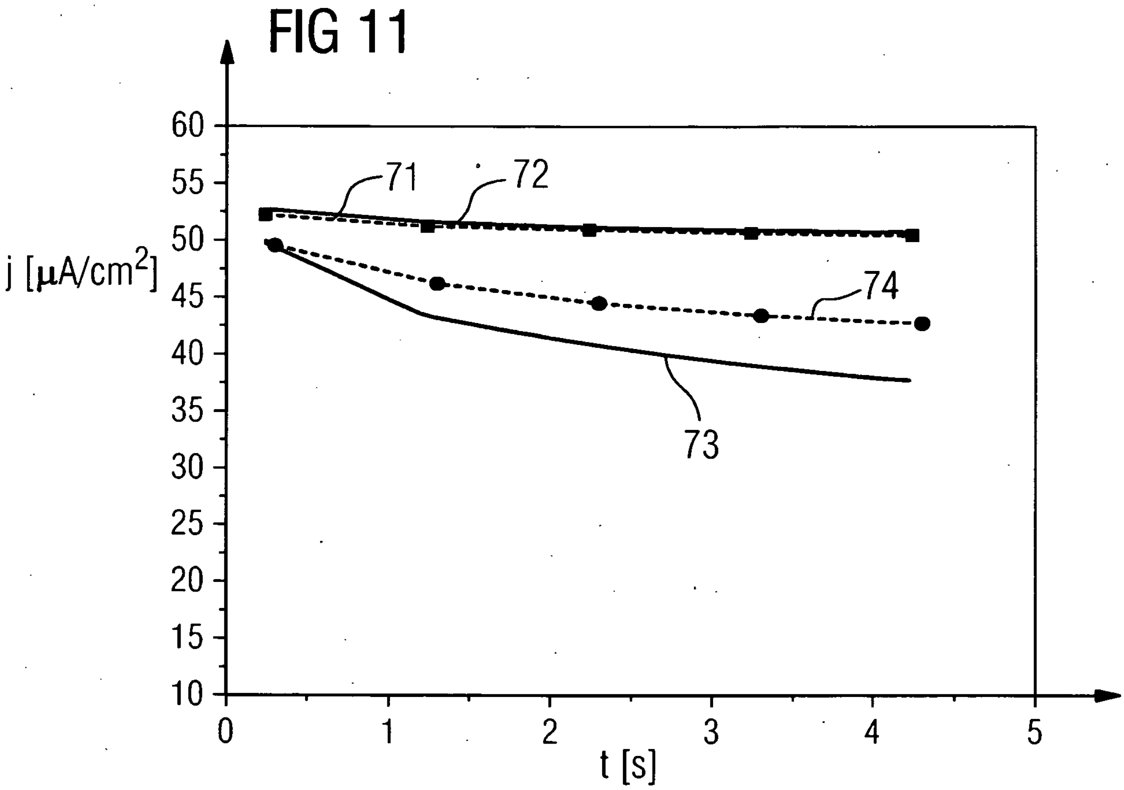
60
55
50
45
40
35
30
25
20
15
10
j [µA/cm²]
71
72
73
74
0
1
2
3
4
5
t [s]

FIG 12

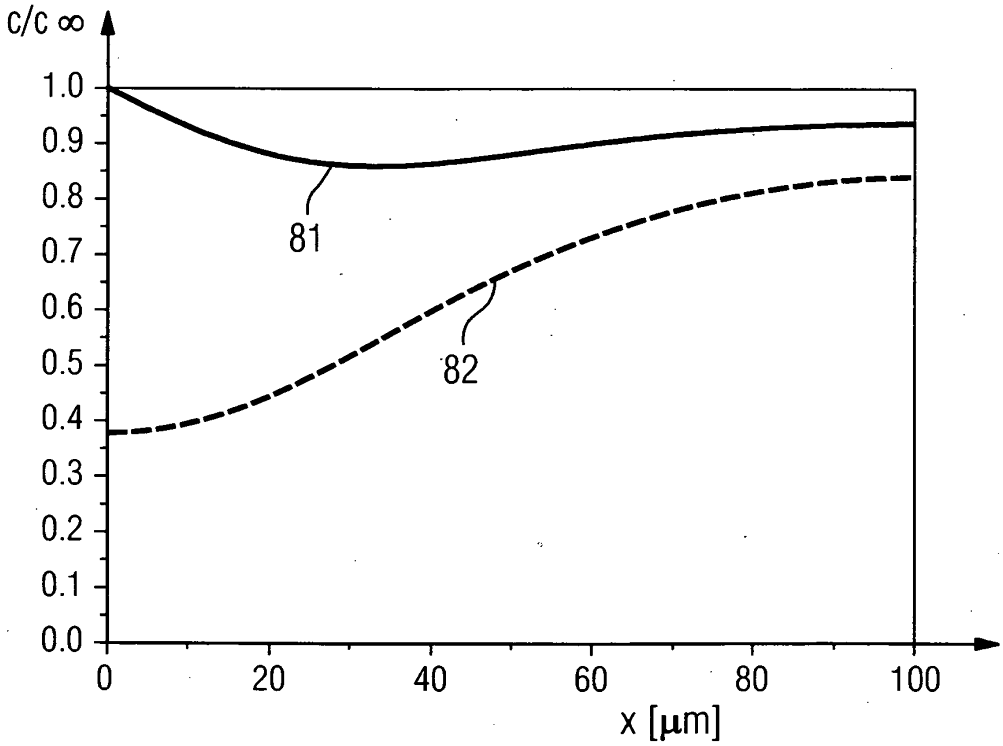
c/c ∞
1.0
0.9
0.8
0.7
0.6
0.5
0.4
0.3
0.2
0.1
0.0
81
82
0
20
40
60
80
100
x [µm]

FIG 13

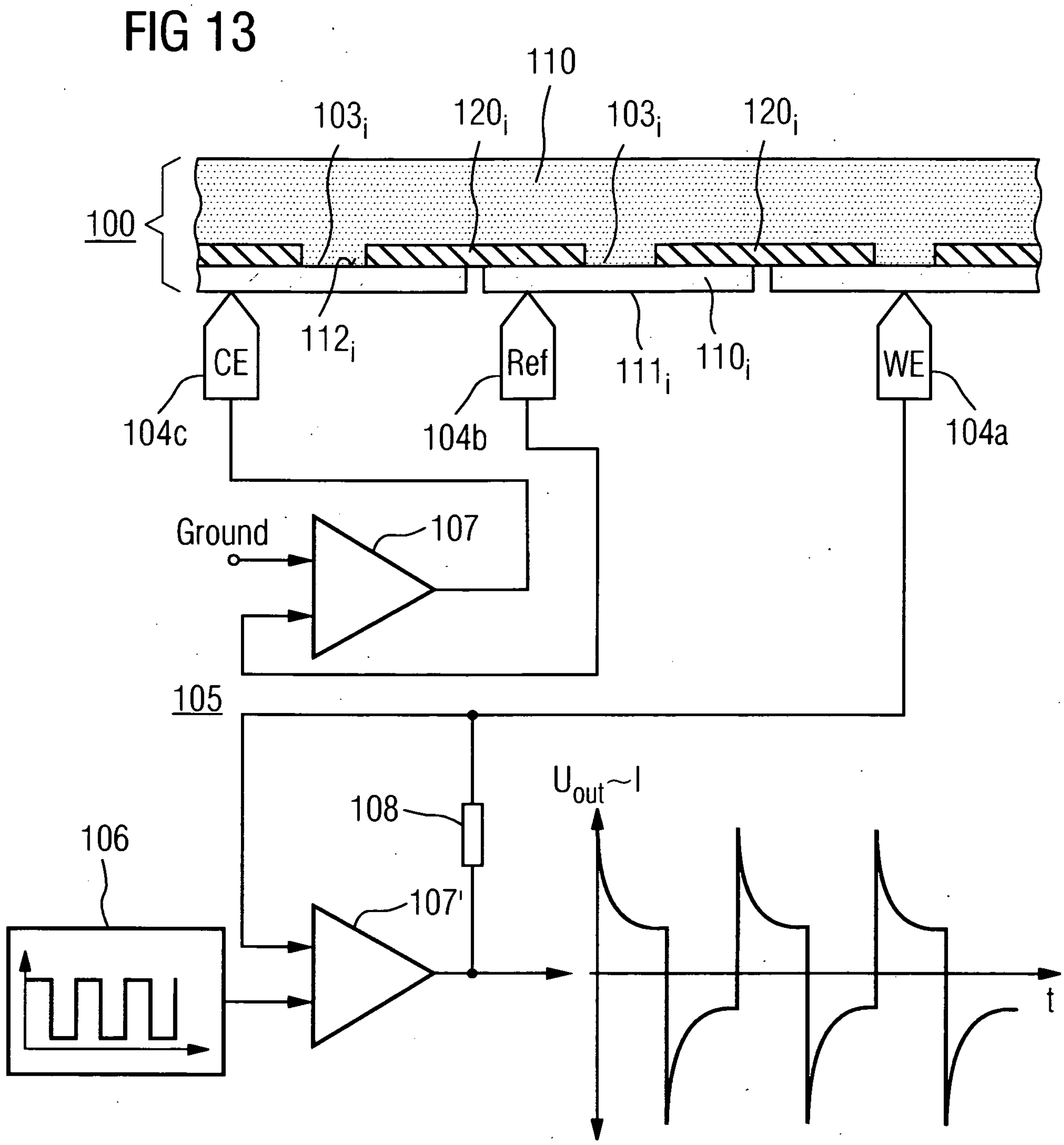
110
$103_i$
$120_i$
$103_i$
$120_i$
100
$112_i$
CE
Ref
$111_i$
$110_i$
WE
104c
104b
104a
Ground
107
105
108
$U_{out}$~I
106
107'
t

FIG 14

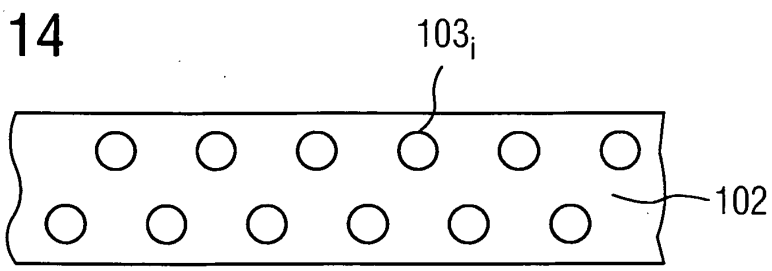
$103_i$
102

FIG 15

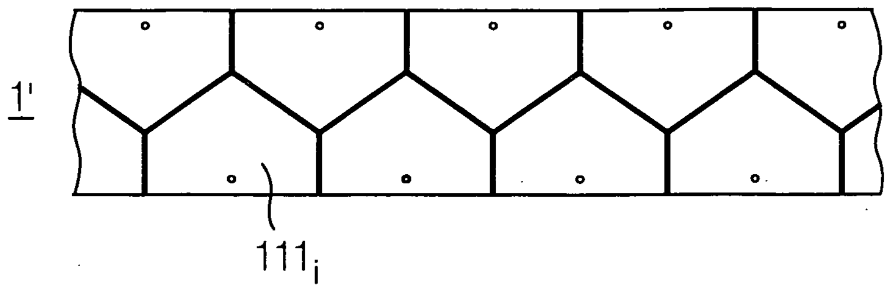
1'
111i

FIG 16

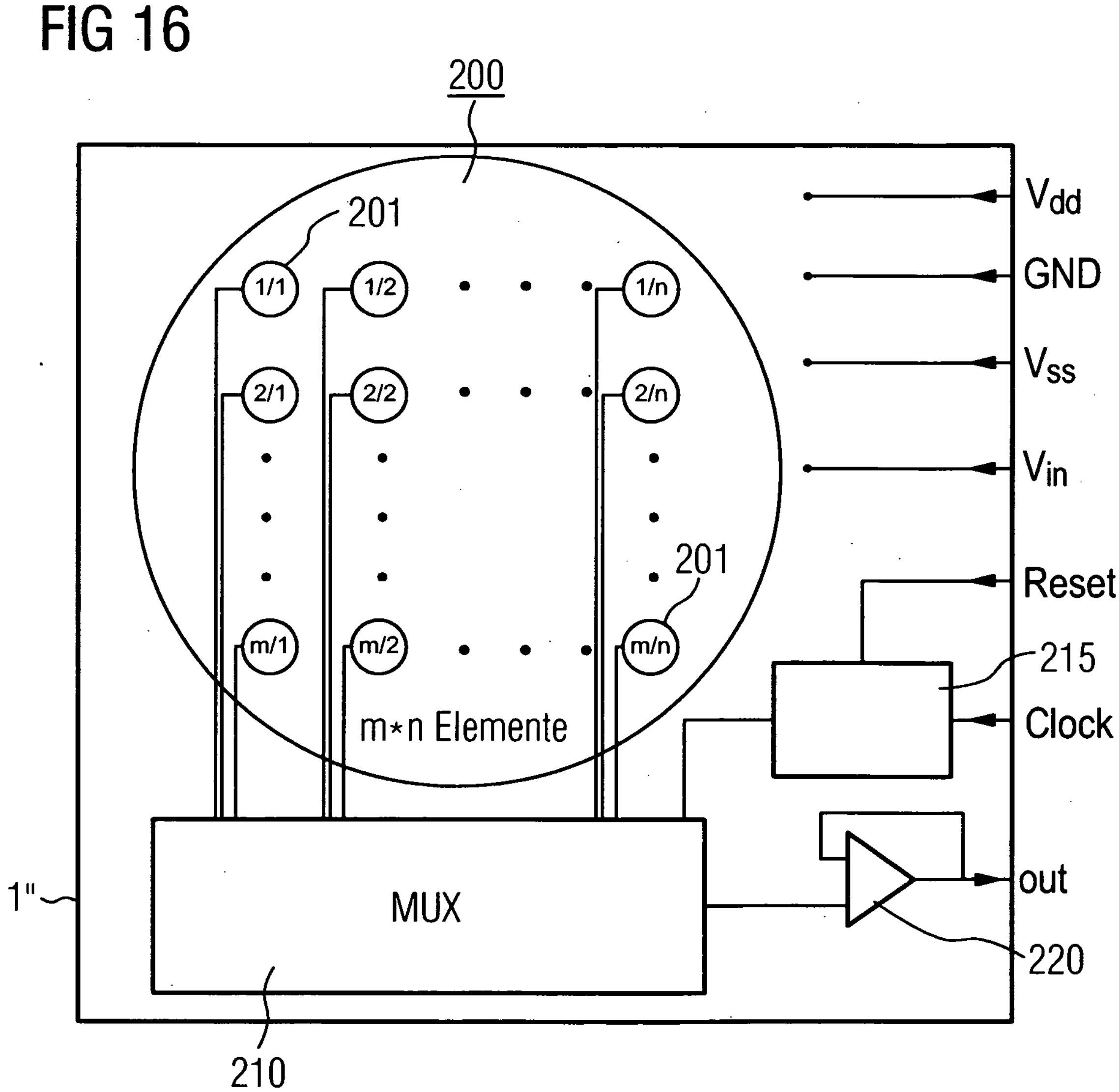
200
201
1/1
1/2
1/n
2/1
2/2
2/n
201
m/1
m/2
m/n
m*n Elemente
Vdd
GND
Vss
Vin
Reset
215
Clock
out
220
MUX
1"
210

FIG 17

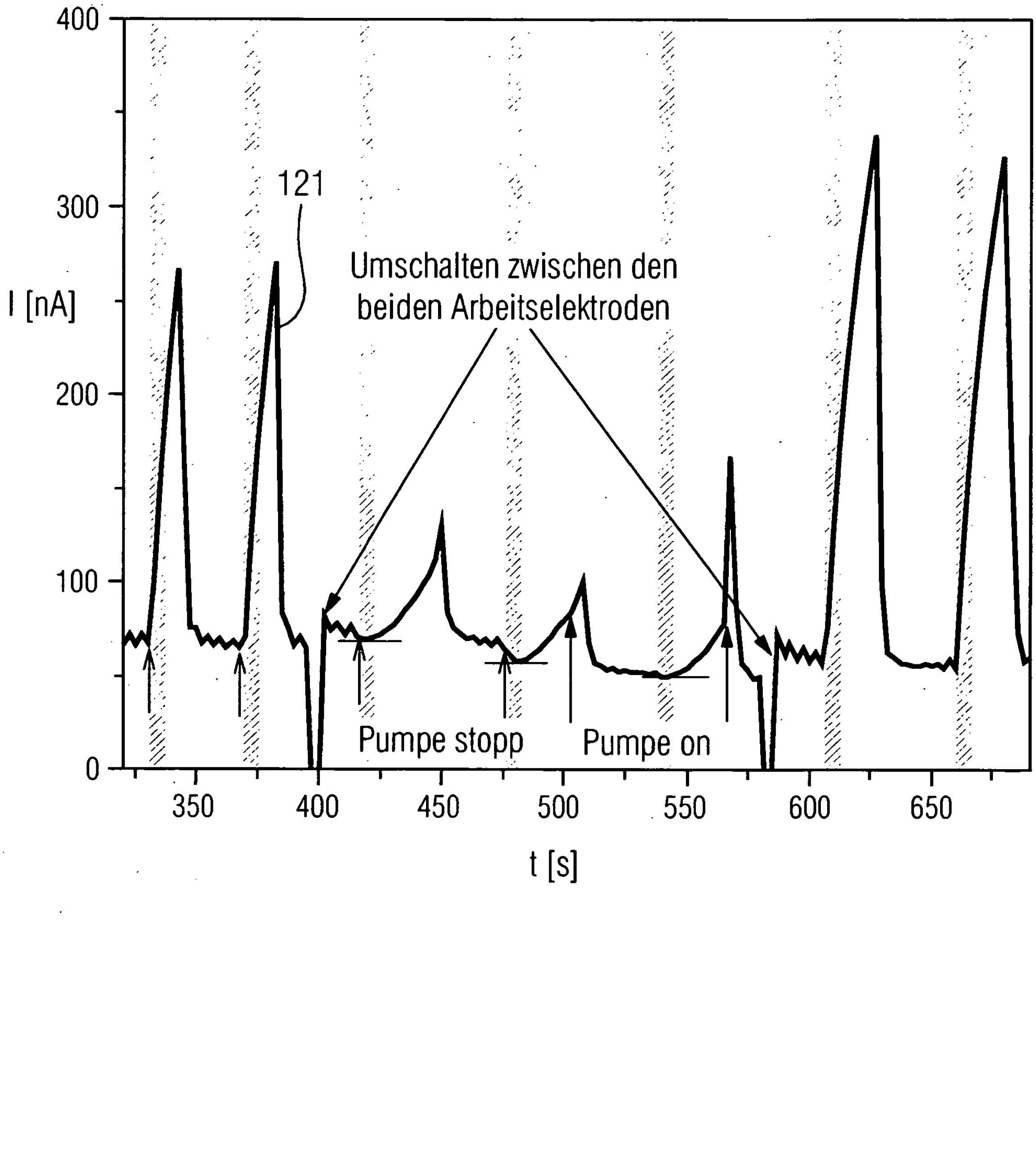
400
300
200
100
0
I [nA]
121
Umschalten zwischen den
beiden Arbeitselektroden
Pumpe stopp
Pumpe on
350
400
450
500
550
600
650
t [s]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6682648 B1
- DE 4335241 A1
- DE 4131731 A1
- DE 19717809 U1
- DE 19917052 A1
- WO 0167587 A1
- DE AZ102004004654952


### In der Beschreibung aufgeführte Nicht-Patentliteratur


- *CLIN. CHEM.,* 1990, vol. 36 (11), 1941-1944
- **K. AOKI.** *J. Electroanal. Chem,* 1989, vol. 270, 35